# EUROPEAN PATENT SPECIFICATION

(11) **EP 2 449 134 B1**
(45) Date of publication and mention of the grant of the patent: **27.07.2016**
(21) Application number: 10747062.7
(22) Date of filing: 22.06.2010
(51) Int. Cl.: C12Q 1/68

(54) **DIAGNOSIS AND TREATMENT OF ALZHEIMER'S DISEASE**
DIAGNOSE UND BEHANDLUNG VON MORBUS ALZHEIMER
DIAGNOSTIC ET TRAITEMENT DE LA MALADIE D ALZHEIMER

(30) Priority: 03.07.2009 GB 0911539; 31.07.2009 GB 0913357; 03.10.2009 GB 0917326
(43) Date of publication of application: 09.05.2012
(60) Divisional application: 15152097.0
(73) Proprietor: University College Cardiff Consultants Limited, Cardiff CF24 0DE (GB)
(72) Inventor: WILLIAMS, Julie, Heath Park Cardiff CF4 4XN (GB); OWEN, Michael, John, Heath Park Cardiff CF4 4XN (GB)
(74) Representative: Fyles, Julie Marie
(86) International application number: PCT/GB2010/001208
(87) International publication number: WO 2011/001135

(56) References cited:
- WO-A2-2006/138716
- WO-A2-2008/085601
- BURDON KATHRYN P ET AL: "Genetic analysis of the clusterin gene in pseudoexfoliation syndrome", MOLECULAR VISION, vol. 14, no. 205-06, September 2008 (2008-09), pages 1727-1736, XP002608021, ISSN: 1090-0535
- DATABASE REfSNP [Online] NCBI; 16 April 2004 (2004-04-16), "Reference SNP Cluster Report rs11136000", XP002608022, Database accession no. rs11136000
- TYCKO B ET AL: "Polymorphisms in the human apolipoprotein-J/clusterin gene: ethnic variation and distribution in Alzheimer's disease.", HUMAN GENETICS OCT 1996 LNKD- PUBMED:8792817, vol. 98, no. 4, October 1996 (1996-10), pages 430-436, XP002608023, ISSN: 0340-6717
- BERTRAM LARS ET AL: "Genome-wide Association Analysis Reveals Putative Alzheimer's Disease Susceptibility Loci in Addition to APOE", AMERICAN JOURNAL OF HUMAN GENETICS, vol. 83, no. 5, November 2008 (2008-11), pages 623-632, XP002608024, ISSN: 0002-9297
- CARRASQUILLO MINERVA M ET AL: "Genetic variation in PCDH11X is associated with susceptibility to late-onset Alzheimer's disease", NATURE GENETICS, vol. 41, no. 2, February 2009 (2009-02), pages 192-198, XP002608025, ISSN: 1061-4036
- LAMBERT JEAN-CHARLES ET AL: "Genome-wide association study identifies variants at CLU and CR1 associated with Alzheimer's disease", NATURE GENETICS, vol. 41, no. 10, October 2009 (2009-10), page 1094, XP002608026, ISSN: 1061-4036
- HAROLD DENISE ET AL: "Genome-wide association study identifies variants at CLU and PICALM associated with Alzheimer's disease", NATURE GENETICS, vol. 41, no. 10, October 2009 (2009-10), page 1088, XP002608027, ISSN: 1061-4036
- DATABASE refSNP [Online] NCBI; 31 October 2002 (2002-10-31), ""Reference SNP Cluster Report: rs3851179", XP002626379, Database accession no. rs3851179
- DATABASE CA [Online] CHEMICAL ABSTRACTS SERVICE, COLUMBUS, OHIO, US; 30 October 2008 (2008-10-30), Kinoshita, Shigeru ET AL: "Method for detection of SNPs in human genes and its use for evaluation of the onset risk of glaucoma", XP002626380, retrieved from STN Database accession no. 149:511008 & WO 2008/130008 A1 (SANTEN PHARMACEUTICAL CO., LTD., JAPAN) 30 October 2008 (2008-10-30)

## Description

The invention relates to the identification of variants in the *CLU*/*APOJ, PICALM, ABCA7, CR1 or BIN1* gene loci or the *MS4A* gene cluster which are novel risk indicators for the development of Alzheimer's disease and also any single nucleotide polymorphisms (SNPs) in linkage disequilibrium therewith; the invention also relates to a method for diagnosing the risk of developing, or the existence of, Alzheimer's disease which involves assaying for the aforementioned variants in the *CLU*/*APOJ, PICALM, ABCA, CR1 or BIN1* gene loci or the *MS4A* gene cluster; and the invention also relates to novel screening tools comprising the aforementioned loci or cluster.

Alzheimer's disease (AD) is the most common form of dementia, it is highly heritable (heritability of up to 76%) but genetically complex. Neuropathologically, the disease is characterised by extracellular senile plaques containing β-amyloid (Aβ) and intracellular neurofibrillary tangles containing hyperphosphorylated τ protein. Four genes have been definitively implicated in its aetiology. Mutations of the *amyloid precursor protein* (*APP*) gene and the *presenilin 1* and *2* genes (*PSEN1, PSEN2*) cause rare, Mendelian forms of the disease, usually with an early-onset. However, in the more common form of AD, only *apolipoprotein E* (*APOE*) has been established unequivocally as a susceptibility gene. Aiming to identify novel AD loci, a small number of genome-wide association studies (GWAS) have been conducted prior to the present study. All have identified strong evidence for association to *APOE,* but less convincing evidence implicating other genes¹⁻⁸. This outcome is consistent with the majority of findings from GWAS of other common phenotypes.

Indeed, the lack of success of the afore GWAS has created a prejudice in the art which teaches away from GWAS as a way of identifying any novel AD loci, other than *APOE.* Despite this thinking we established a collaborative consortium from Europe and the USA from which we were able to draw upon a combined sample of up to 19,000 subjects (before quality control) and conducted a two-stage study. In Stage 1, 14,639 subjects were genotyped on Illumina platforms. 5,715 samples were genotyped for the present study using the Illumina 610-quadchip⁹; genotypes for the remaining subjects were either made available to us from population control datasets or through collaboration and were genotyped on the Illumina HumanHap550 or the HumanHap300 BeadChips. Prior to association analysis, all samples and genotypes underwent stringent quality control, which resulted in the elimination of 53,383 autosomal SNPs and 2,850 subjects. Thus, in Stage 1, we tested 529,205 autosomal SNPs for association in up to 11,789 subjects (3,941 AD cases, 7,848 controls of which 2,078 were elderly screened controls, see Table S1).

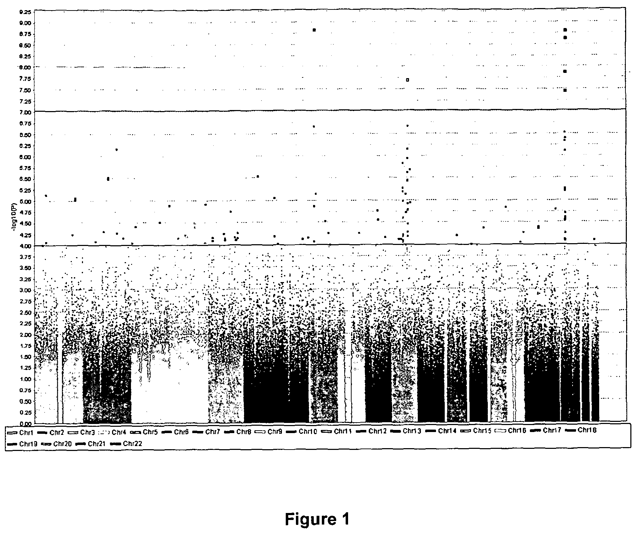

In addition to the known association with the *APOE* locus, GWA analysis identified two novel loci at a genome-wide level of significance (see Figure 1).

One of the novel SNPs, rs11136000, is located within an intron of *clusterin (CLU,* also known as *APOJ*) on chromosome 8 (p= 1.4x10⁻⁹, OR=0.840); the other SNP, rs3851179, is 88.5 kb 5' to *phosphatidylinositol-binding clathrin assembly* (*PICALM*) on chromosome 11 (p = 1.9x10⁻⁸, OR=0.849). Notably, therefore, neither SNP maps to a coding site and so is responsible for changes in an identified protein. They may therefore produce their effect at the level of gene expression, temporally or spatially, which, in turn, may affect transcription levels.

The afore statistical data provides compelling evidence that the two novel loci identified herein and their corresponding SNPs associate, with extremely high levels of probability, with Alzheimer's disease. This means that these SNPs can be used as powerful predicative tools for diagnosing the likelihood of developing Alzheimer's disease or the existence of Alzheimer's disease in the early stages of the condition.

A stage 2 sample (2,023 AD cases, 2,340 controls) provided further support for the association of these novel loci (*CLU* combined p= 8.5x10⁻¹⁰, OR=0.86; *PICALM* combined p= 1.3x10⁻⁹, OR=0.86).

In a preliminary attempt to attribute the source of the association to a functional variant, we used publicly available data to identify additional SNPs at each locus that were correlated through linkage disequilibrium (LD) with either of the afore novel SNPs or that might plausibly have functional effects. These markers were genotyped through the stage 2 sample. A synonymous SNP (rs7982) in the *CLU* gene was in strong LD (r²=0.95) with the genome-wide significant (GWS) SNP and showed a similar level of evidence for association with AD in the whole sample (meta-p =8x10⁻¹⁰; stage 1 genotypes were imputed). This SNP is in exon 5 of the *CLU* gene, which codes for part of the beta chain of the protein and may influence a predicted exon splicing enhancer. Several potentially functional SNPs were identified at the *PICALM* locus. Of these, two showed good evidence for association; rs561655, which is within a putative transcription factor binding site and rs592297, which is a synonymous SNP in exon 5 of the gene that may influence a predicted exon splicing enhancer.

The predominant form of clusterin is a secreted heterodimeric glycoprotein of 75-80kDa. The single copy gene spans about 16kb on chromosome 8p21-p12 and encodes an mRNA of approximately 2kb that translates into a 449 amino acid primary polypeptide chain. *Clusterin* is expressed in all mammalian tissues and there is strong evidence that *clusterin* levels are elevated in a number of pathological conditions involving injury or chronic inflammation of the brain. In Alzheimer's disease brain, *clusterin* expression is reported to be increased in affected cortical areas and is present in amyloid plaques and in the cerebrospinal fluid of AD cases.

Clusterin is a multi-functional molecule. It interacts with the soluble form of Aβ in animal models of disease and binds soluble Aβ in a specific and reversible manner, forming complexes that have been shown to cross the blood-brain barrier. Interestingly, ApoE also appears to act as a molecular chaperone for Aβ and influences when it aggregates and deposits as well as influencing Aβ conformation and toxicity. In a similar way, clusterin appears to regulate both the toxicity and conversion of Aβ into insoluble forms. Furthermore, ApoE and clusterin have been shown to cooperate in suppressing Aβ deposition and ApoE and clusterin may critically modify Aβ clearance at the blood brain barrier, suggesting a role for clusterin in the amyloidogenic pathway. Levels of ApoE protein appear to be proportional to *APOE-*ε4 allele dose levels, *i.e.* expression levels are reduced in ε4 homozygotes compared with heterozygotes. Conversely, clusterin levels are increased in proportion to *APOE-*ε4 allele dose suggesting an induction of clusterin in individuals with low ApoE levels. Thus, the strong statistical evidence for the involvement of this gene in AD has additional support in terms of biological functionality.

The second gene locus to show compelling evidence for association with AD is *PICALM* (phosphatidylinositol-binding clathrin assembly protein; also known as *CALM:* clathrin assembly lyphoid-myeloid leukaemia gene). *PICALM* is ubiquitously expressed in all tissue types with prominent expression in neurons, where it is non-selectively distributed at the pre- and post synaptic structures. It has been shown that PICALM is involved in clathrin-mediated endocytosis (CME), an essential step in the intracellular trafficking of proteins and lipids such as nutrients, growth factors and neurotransmitters. Of relevance to AD, PICALM appears to be involved in directing the trafficking of VAMP2. VAMP2 is a SNARE protein that plays a prominent role in the fusion of synaptic vesicles to the presynaptic membrane in neurotransmitter release, a process that is crucial to neuronal function. AD brains show a reduced number of synapses and stereological and biochemical analysis has shown that this reduction in synaptic density correlates with cognitive defects better than the accumulation of plaques. More recent analysis indicates synapses within AD brains may be dysfunctional even before they visibly degenerate. Therefore, we can hypothesise that genetically directed changes in PICALM function result in perturbations at the synapse, possibly through synaptic vesicle cycling, thereby increasing risk for AD. Alternatively, PICALM could influence risk of AD through APP processing via endocytic pathways resulting in changes in Aβ levels. Cell culture experiments have shown that full length APP is retrieved from the cell surface by CME and inhibition of endocytosis reduces APP internalisation and reduces Aβ production and release. Increased synaptic activity is known to lead to the elevated endocytosis of synaptic vesicle proteins and Cirrito *et al.* have since provided evidence *in vivo* that the increased CME, triggered by increased synaptic activity, drives more APP into endocytotic compartments resulting in an increase of Aβ production and release. Thus, as for clusterin, the strong statistical evidence for the involvement of PICALM in AD has support in terms of biological functionality.

We also tested whether the observed number of significant associations observed in the GWAS exceeded what would be expected by chance. Having removed SNPs within the *APOE, CLU* and *PICALM* loci (see Methods) we focused on those which showed most evidence for association (p< 1x10⁻⁵). Approximately 13 independent signals were observed; less than 4 would be expected by chance (p= 7.5x10⁻⁶). Table 2 shows the loci implicated and provides strong evidence for association with the complement receptor 1 (CR1) gene. Also noteworthy is the bridging integrator 1 (*BIN1*) gene, which produces a protein involved in synaptic vesicle endocytosis. These data thus provide strong evidence that these genes are associated with AD.

We sought to test those SNPs which showed the most promising evidence for association with AD in an independent sample comprising 3,262 AD cases and 5,064 controls which included the stage 2 samples previously discussed⁹. SNPs that showed *P<1* x 10⁻⁵ from a meta-analysis of four GWAS datasets (a combined sample of up to 6,978 cases, 13,903 controls) were selected for genotyping (see Methods). For SNPs that showed evidence for association in the independent sample (P<0.05) additional consortia were approached so to combine all available AD GWAS data in an inverse variance weighted meta-analysis (see Methods). Four SNPs showed GWS evidence for association with AD (see Table 3); rs3764650 within *ABCA7* (*P*=7.3 x 10⁻¹⁰, OR= 1.22), rs670139 at the *MS4A* gene cluster (P=1.2 x 10⁻⁹, OR= 1.12), rs744373 at the *BIN1* locus (*P*=2.1x10⁻¹², OR =1.14) and rs3818361 at the *CR1* locus (*P*=1.9 x 10⁻¹², OR= 1.17)¹⁰

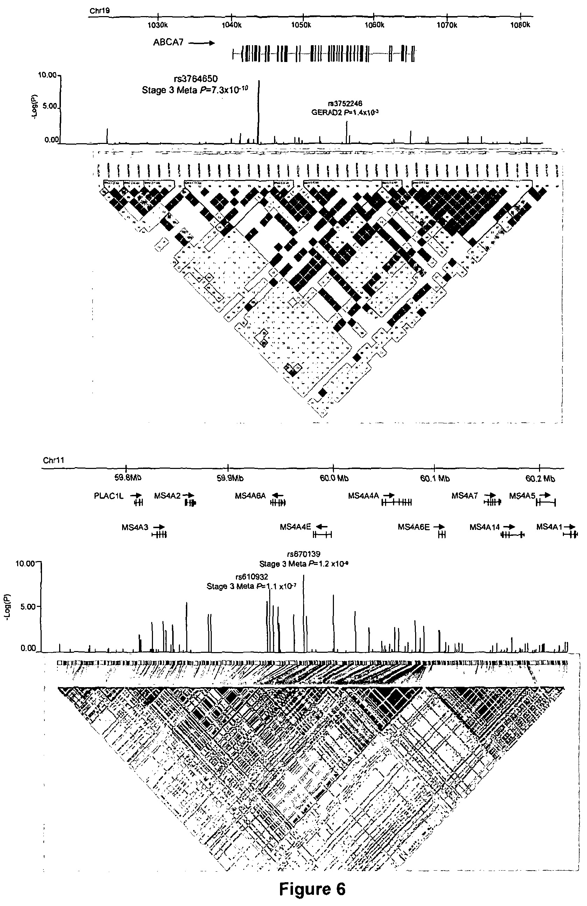

Variant rs3764650 is located in intron 13 of the *ATP-binding cassette, sub-family A, member 7* (*ABCA7*) gene (Figure 6). *ABCA7* encodes an ATP-binding cassette (ABC) transporter: the ABC transporter superfamily has roles in transporting a wide range of substrates across cell membranes. *ABCA7* is highly expressed in brain, particularly in hippocampal CA1 neurons and in microglia. ABCA7 is involved in the efflux of lipids from cells to lipoprotein particles: the main lipoprotein in brain is APOE followed by CLU, thus ABCA7 may have a role in modulating their effects, although no evidence for genetic interactions was observed here (see Table S13). In addition ABCA7 has been shown to regulate APP processing and inhibit β-amyloid secretion in cultured cells overexpressing *AP.*

The genes in the *MS4A* cluster on chromosome 11 (chr11: 59,570,863-59,863,681 (Build GRCh37/hg19)) (Figure 6) have a common genomic structure with all other members of the family, including transmembrane domains, indicating that they are likely to be part of a family of cell surface proteins. MS4A2 encodes the beta subunit of high affinity IgE receptors. The remaining genes in the LD block, including *PLAC1L* have no known specific functions.

BIN1 (also known as AMPH2, amphiphysin isoform 2) along with PICALM, functions in receptor-mediated endocytosis (RME). BIN1 expression is not brain specific, but there are several isoforms with enriched expression in brain. Knockdown of mammalian *BIN1* delays endosome recycling and deletion mutants of the single amphiphysin isoform (AMPH1/AMPH2) orthologue in C. elegans (Amph-1) show defective endosome recycling.

CR1 is predominantly involved in adaptive immunity and is abundantly expressed on red blood cells, especially on intravascular erythrocytes and has been detected on neurons. CR1 mediates cellular binding to particles and immune complexes where it recognises that complement has been activated. CR1 can act as a negative regulator of the complement cascade, mediate immune adherence and phagocytosis and inhibit both the classical and alternative complement pathways. Markers of inflammation have been associated with AD previously and inflammatory processes proposed as pathogenic contributors. These data showing the involvement of several putative inflammatory genes (*CLU, CR1, ABCA7 and* possibly genes in the *MS4A* cluster) in AD, support a primary role for inflammatory processes in disease development.

### Statements of the Invention

According to a first aspect of the invention there is provided a method for screening for, or diagnosing the likelihood of developing, or the existence of, Alzheimer's disease comprising:
(a) providing a tissue sample which has been extracted from a human body of an individual to be tested wherein the tissue sample contains at least a locus containing the *PICALM gene* on chromosome 11;
(b) examining said locus in order to identify whether SNP rs3851179 is present; and
(c) where SNP rs3851179 is present concluding that the individual from whom the sample has been extracted is likely to develop, or is suffering from, Alzheimer's disease.

Additionally, or alternatively either of the above methods may be practised by substituting reference to the *PICALM* gene on chromosome 11 for either the *complement*
*receptor 1* gene (*CR1*) on chromosome 1, the *bridging integrator 1* gene (*BIN1*) on chromosome 2, *the ATP-binding cassette, sub-family A, member* 7 *(ABCA7)* on chromosome 19 or the chromosome 11 *membrane-spanning 4A* (*MS4A*) gene cluster and examining said loci to see if any one of the
following SNPs is present rs1408077, rs6701713 or rs3818361 (for *CR1*); rs7561528 or rs744373 (for *BIN1*); rs3764650 (for *ABCA7*)*,* and rs670139, rs610932, rs676309, rs667897, rs662196, rs583791 or rs1562990 for the *MS4A* gene cluster; and where the said SNP is present concluding that the individual from whom the sample has been extracted is likely to develop, or is suffering from, Alzheimer's disease.

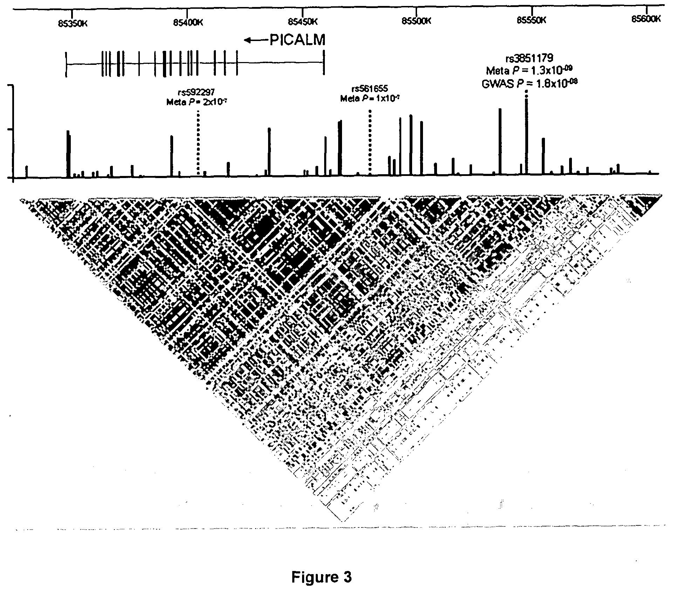

In any of the afore aspects of the invention reference to SNP rs3851179 is reference to a T to C base change 85.5kb upstream of the *PICALM* gene (transcript variant 1; NM_007166.2; transcript variant 2; NM_001008660.1) at position chr11 :
chr11:85546288-85546288 (Build NCBI36/hg18) (See Table S16). The mapping of this SNP is shown in Figure 3.

In any of the afore aspects of the invention reference to SNP rs1408077 is reference to a base change of C to A in intron 38 of the of the *CR1* gene (transcript variant F; NM_000573.3) at position chr1:205870764-205870764 (Build NCBI36/hg18) (See Table S16).

In any of the afore aspects of the invention reference to SNP rs6701713 is reference to a base change of G to A in intron 31 of the of the *CR1* gene (transcript variant F; NM_000573.3) at position chr1:205852912-205852912 of the (Build NCBI36/hg18) (See Table S16).

In any of the afore aspects of the invention reference to SNP rs3818361 is reference to a base change of G to A in intron 29 of the of the *CR1* gene (transcript variant F; NM_000573.3) at position chr1:205851591-205851591 (Build NCBI36/hg18) (See Table S16).

In any of the afore aspects of the invention reference to SNP rs7561528 is reference to a base change of G to A 24.8kb upstream of the *BIN1* gene (transcript variant 1; NM_139343.1; transcript variant 6; NM_139348.1; transcript variant 10; NM_139351.1; transcript variant 2; NM_ 139344.1; transcript variant 3; NM_139345.1; transcript variant 8; NM_004305.2; transcript variant 9; NM_139350.1; transcript variant 7; NM_139349.1; transcript variant 5; NM_ 139347.1; transcript variant 4; NM_139346.1) at position chr2: 127606107-127606107 (Build NCBI36/hg18) (See Table S16).

In any of the afore aspects of the invention reference to SNP rs744373 is reference to a base change of A to G 29.8kb upstream of the *BIN1* gene (transcript variant 1; NM_139343.1; transcript variant 6; NM_139348.1; transcript variant 10; NM_139351.1; transcript variant 2; NM_139344.1; transcript variant 3; NM_139345.1; transcript variant 8; NM_ 004305.2; transcript variant 9; NM_ 139350.1; transcript variant 7; NM_139349.1; transcript variant 5; NM_ 139347.1; transcript variant 4; NM_139346.1) at position chr2:127611085-127611085 (Build NCBI36/hg18) (See Table S16).

In any of the afore aspects of the invention reference to SNP rs3764650 is reference to a base change of T to G in intron 13 of the of *the ABCA7gene* (N_019112.3) at position chr19:1046520-1046520 (Build GRCh37/hg19) (See Table S16). The mapping of this SNP is shown in Figure 6.

In any of the afore aspects of the invention reference to SNP rs1562990 is reference to a base change of C to A 25 kb upstream of the *MS4A4A* gene (transcript variant 2; NM_148975.1) at position chr11:60023087-60023087 (Build GRCh37/hg19) (See Table S16).

In any of the afore aspects of the invention reference to SNP rs667897 is reference to a base change of A to G 2 kb upstream of the *MS4A6A* gene (transcript variant 1; NM_152852.1; transcript variant 3; NM_152851.1) at position chr11:59936979-59936979 (Build GRCh37/hg19) (See Table S16).

In any of the afore aspects of the invention reference to SNP rs676309 is reference to a base change of A to G 4 kb upstream of the *MS4A4E* gene at position chr11:60001573-60001573 (Build GRCh37/hg19) (See Table S16).

In any of the afore aspects of the invention reference to SNP rs583791 is reference to a base change of G to A in intron 3 of the *MS4A6A* gene (transcripts 1; NM_152852.1; transcript 2; NM_022349.2; transcript 3; NM_152851.1) at position chr11:59947252-59847252 (Build GRCh37/hg19) (See Table S16).

In any of the afore aspects of the invention reference to SNP rs662196 is reference to a base change of G to A in intron 5 of the *MS4A6A* gene (transcripts 1; NM_152852.1; transcript 2; NM_022349.2; transcript 3; NM_152851.1) at position chr11:59942757-59942757 (Build GRCh37/hg19) (See Table S16).

In any of the afore aspects of the invention reference to SNP rs610932 is reference to a base change of A to C in the 3 prime untranslated region of the *MS4A6A* gene (transcripts 1; NM_152852.1 ; transcript 2; NM_022349.2) at position chr11:59939307-59939307 (Build GRCh37/hg19) (See Table S16). The mapping of this SNP is shown in Figure 6.

In any of the afore aspects of the invention reference to SNP rs670139 is reference to a base change of C to A 21 kb upstream of the *MS4A4E* gene at position chr11: 59971795- 59971795 (Build GRCh37/hg19) (See Table S16). The mapping of this SNP is shown in Figure 6.

In any of the afore aspects of the invention the said methodology may be undertaken using any one or more of the following variants, including any combination thereof:
SNP rs3851179; SNP rs1408077; SNP rs6701713; SNP rs3818361; SNP rs7561528; SNP rs744373; SNP rs561655; SNP rs592297; SNP rs3764650; SNP rs1562990; SNP rs667897; SNP rs676309; SNP rs583791; SNP rs662196; SNP rs670139 and SNP rs610932.

Preferably, in any of the above aspects of the invention identifying either one, or more, of the said SNPs involves the use of conventional genetic screening techniques well known to those skilled in the art of genotyping. For example, and without limitation, the aforementioned methods may be practised by the use of suitably labelled oligonucleotides which upon binding to, and so detecting the SNP of interest, emit a detectable signal representative of the presence of said SNP. Methods for the design of such oligonucleotides are well known to those skilled in the art and routinely practised in the identification and labelling of DNA. Further, the information provided in Table S16 enables those skilled in the art to design such oligonucleotides in conventional manner.

In either of the aforementioned aspects of the invention said tissue sample may be amplified prior to performing step (b) above. More preferably still, said amplified tissue may be enzymatically fragmented prior to performing step (b) above.

In yet a further preferred method of the invention said complementary oligonucleotide may be attached or bound to a solid phase or substrate and said, optionally, amplified and fragmented tissue sample may be exposed to said solid phase prior to performing the detection step referred to in (b) above.

According to the disclosure there is provided a kit for performing any one or more of the aforementioned methods of the invention wherein the kit comprises at least one oligonucleotide that is complementary to least one of the aforementioned loci and which is either provided with a suitable label that emits a detectable signal upon binding to the SNP of interest or there is provided associated labelling means which can be used in combination with said oligonucleotide whereby binding of the oligonucleotide to the SNP of interest enables the labelling means to be used to detect the aforementioned binding and so produce a signal representative of the presence of said SNP.

In a preferred disclosure said oligonucleotide(s) is/are immobilised on a solid support such as, without limitation, a bead, array or substrate.

According to a further aspect of the invention there is provided a method for screening for, or diagnosing the likelihood of developing, or the existence of, Alzheimer's disease comprising:
(a) providing a tissue sample which has been extracted from a human body of an individual to be tested wherein the tissue sample contains at least a locus containing the *PICALM* gene on chromosome 11;
(b) examining said locus in order to identify whether SNP rs3851179 and/or SNP rs561655 or rs592297 which is in linkage disequilibrium therewith is present; and
(c) where any one or more of the said SNPs is present concluding that the individual from which the sample has been extracted is likely to develop, or is suffering from, Alzheimer's disease.

In any of the afore aspect of the invention reference to SNP rs561655 is reference to a base change of A to G 20.2kb upstream of the *PICALM* gene (transcript variant 1; NM_007166.2) at position chr11:85477927-85477927 of the March 2006 human reference sequence (NCBI Build 36.1) (See Table S16)..

In any of the above aspect of the invention reference to SNP rs592297 is reference to a base change of C to T in exon 5 of the *PICALM gene* (transcript variant 1; NM_007166.2) at position chr11:85403585-85403585 of the March 2006 human reference sequence (NCBI Build 36.1) (See Table S16)..

According to a the disclosure there is provided a nucleic acid molecule comprising any one or more of the following loci or cluster: *CLU*/*APOJ, PICALM, ABCA7, CR1 or BIN1, MS4A* including one or more of the following variants, including any combination thereof;
SNP rs1136000; SNP rs3851179; SNP rs1408077; SNP rs6701713; SNP rs3818361; SNP rs7561528; SNP rs744373; SNP rs7982; SNP rs561655; SNP rs592297; SNP rs3764650; SNP rs1562990; SNP rs667897; SNP rs676309; SNP rs583791; SNP rs662196; SNP rs670139 and SNP rs610932.

According to the disclosure there is provided a research tool for identifying therapeutics, labelling or identification means comprising: at least one genetic locus wherein said locus comprises on or more of the loci referred to above, that is, *CLU, PICALM, CR1, BIN1, ABCA7,* or *MS4A* loci which associate with AD, and more preferably the SNPs mentioned above in connection therewith.

According to the disclsoure there is provided a cell or cell-line comprising one or more of the genetic loci of the invention and one or more of the associated SNPs for use to test whether a potential therapeutic, label or identification means can be used to treat AD or label or identify a marker that associates with AD.

Most suitably the cell or cell line comprises a funcctional endocytic, apoptotic, complement or innate immune response pathway whose modulation, by a test substance, can be used to determine the efficacy of said substance.

In the claims which follow and in the preceding description of the invention, except where the context requires otherwise due to express language or necessary implication, the word "comprises", or variations such as "comprises" or "comprising" is used in an inclusive sense i.e. to specify the presence of the stated features but not to preclude the presence or addition of further features in various embodiments of the invention.

Other features of the present invention will become apparent from the following examples.

Moreover, unless stated otherwise, any feature disclosed herein may be replaced by an alternative feature serving the same or a similar purpose.

The invention will now be exemplified with reference to the following Accession numbers and Figures wherein:
**Accession numbers**
   GenBank: *CLU isoform* 1 mRNA, NM_001831.2; *CLU isoform* 2 mRNA, NM_203339.1 ; *CLU* isoform 3 mRNA, NM_001 171138.1; *PICALM isoform* 1 mRNA, NM_007166.2; *PICALM* isoform 2 mRNA, NM_001008660.1. *CR1* isoform S mRNA, NM_000651.4; *CR1* isoform F mRNA, NM_000573.3; *BIN1* isoform 6 mRNA, NM_ 139348.1; *BIN1* isoform 10 mRNA, NM_139351.1; *BIN1* isoform 2 mRNA, NM_ 139344.1; *BIN1* isoform 3 mRNA, NM_139345.1; *BIN1* isoform 8 mRNA, NM_ 004305.2; *BIN1* isoform 1 mRNA, NM_139343.1; *BIN1* isoform 9 mRNA, NM_ 139350.1; *BIN1* isoform 7 mRNA, NM_139349.1; *BIN1* isoform 5 mRNA, NM_ 139347.1; *BIN1* isoform 4 mRNA, NM_139346.1; *ABCA7* mRNA, NM_01 9112.3; *PLAC1L* mRNA, NM_173801.3; *MS4A6E* mRNA NM_139249.2; *MS4A3* isoform 2 mRNA, N_001031809.1; *MS4A3* isoform 1 mRNA, NM_ 006138.4; *MS4A3* isoform 3 mRNA, NM 001031666.1; *MS4A2* isoform 2 mRNA, N_001142303.1; *MS4A2 isoform* 1 mRNA, NM_000139.3; *MS4A6A* isoform 1 mRNA, NM_152852.1; *MS4A6A* isoform 3 mRNA, NM_152851.1; *MS4A6A* isoform 2 mRNA, NM_022349.2; *MS4A4A* isoform 2 mRNA, NM_148975.1; *MS4A4A* isoform 1 mRNA, NM_024021.2.
**Figure 1****.** Scatterplot of chromosomal position (x-axis) against -log 10 GWAS P-value (y-axis). The y-axis scale has been limited to 9.25 (p = 5.6x10⁻¹⁰), although highly significant association was observed with SNPs in the vicinity of the *APOE* locus *(e.g.* rs2075650 with p = 1.8x10⁻¹⁵⁷). The threshold for genome-wide significance (p ≤ 9.4x10⁻⁸) is indicated by the red horizontal line. 761 SNPs with p≤ 1x10⁻³ lie above the blue horizontal line. The plot was produced using Haploview v4.0. (http://www.broad.mit.edu/mpg/haploview/) ;

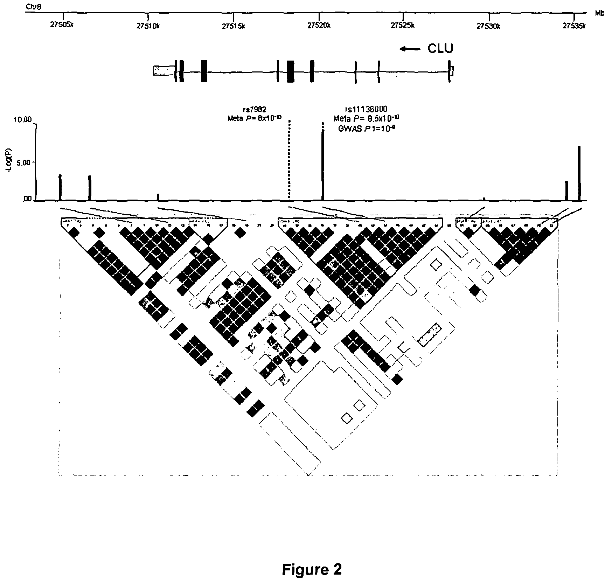

**Figure 2****.** is a schematic of the *CLU* gene based on *CLU* transcript variant 1. Chromosome position in megabases is shown at the top of the diagram. Gene schematic; horizontal arrow indicates direction of transcription, black boxes indicate gene exons, grey boxes indicate UTR, blue boxes indicate transcript variant 2 specific UTR. The -log₁₀(P) of the SNPs genotyped in the GWA study is shown in chart graph with GWS SNP rs11136000 highlighted in red. Meta p-values are indicated by dotted blue lines for GWS SNPs rs11136000 and rs7982. The D' LD block structure of the *CLU* gene and surrounding region (chr8: 27,502,817-27,535,944), according to the CEU HapMap data, is provided at the bottom of the figure with lines indicating where each genotyped SNP is represented. Note, *CLU* transcript variant 2 does not contain exon 1 or the 5'UTR of transcript variant 1, also, in transcript variant 2, half of exon 2 is UTR;
**Figure 3** is a schematic of the *PICALM* gene based on *PICALM* transcript variant 1. Chromosome position in megabases is shown at the top of the diagram. Gene schematic; horizontal arrow indicates direction of transcription, black boxes indicate gene exons, grey boxes indicate UTR, blue boxes indicates a specific exon for transcript variant 2. The -log₁₀(P) of the SNPs genotyped in the GWA study is shown in chart graph with the GWS SNP rs3851179 highlighted in red. Meta p-values are indicated by dotted blue line for GWS SNP (rs3851179) plus putative functional variants (rs592297 and rs561655) that approach genome-wide significance. The D' LD block structure of the *PICALM gene* and surrounding region (char11: 85,326,133-85,605,600), according to the CEU HapMap data, is provided at the bottom of the figure;

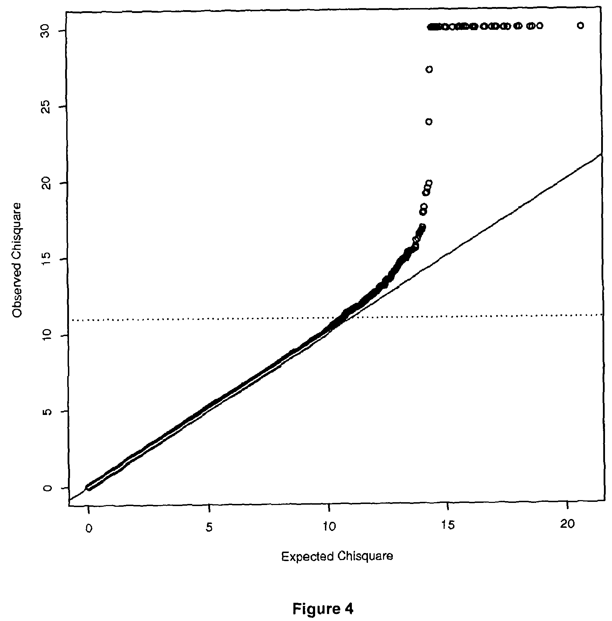

**Figure 4** shows an example of quantile-quantile plot when comparing different sets of controls. In this example, screened controls from the UK and Ireland, genotyped on the Illumina 610-quadchip are compared with controls from the 1958 British birth cohort, genotyped on the Illumina HumanHap550. The observed association χ² test statistics (y-axis) have been plotted against those expected under the null expectation (x-axis). The y-axis was limited at 30 although higher values were observed. The line of equality is coloured red. An exclusion χ² threshold of 11 was employed (indicated by the dotted horizontal line);

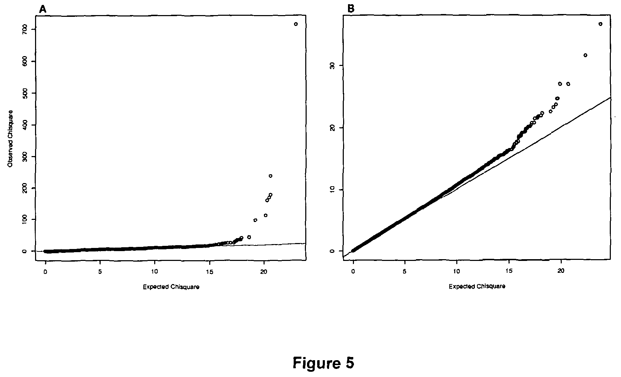

**Figure 5** shows an quantile-quantile (Q-Q) plot of 529,205 observed genome-wide association χ² test statistics (y-axis) against those expected under the null expectation (x-axis). The line of equality is coloured red. λ=1.037. B) Q-Q plot after removal of 170 SNPs at the *APOE* locus. 529,035 SNPs remain (λ=1.037); and
**Figure 6** shows a schematic of the associated variants reported in reference to (a) the *ABCA7* gene and (b) chromosomal region chr11:59.81 Mb-60.1 Mb harbouring members of the *MS4A* gene cluster. Chromosome positions are shown at the top of the schematics (Build GRCh37/hg19). Gene schematic: horizontal arrows indicate directions of transcription, black boxes indicate gene exons/UTR. The -Log₁₀(*P*) of the SNPs analyzed in Stage 1 are shown in chart graph. The Stage 3 inverse variance weighted meta-analysis P-values for SNPs rs3764650 (*ABCA7*), rs610932 (*MS4A6A*) and rs670139 (*MS4A4E*) are indicated by the red lines. The non-synonymous SNP rs3752246 at the *ABCA7* locus genotyped in the GERAD2 sample is indicated by the blue line (D'=0.89, r²=0.36 with rs3764650). The D' LD block structure of the *ABCA7* gene plus surrounding region, and chr11:59.81 Mb-60.1 Mb according to the CEPH HapMap data, are provided at the bottom of each schematic with lines indicating where each SNP genotyped on the Illumina 610-quad chip is represented.

### Materials and Methods

### Sample ascertainment and diagnostic criteria

The study comprised a Stage 1 'discovery sample' of 4,957 AD cases and 9,682 controls and a Stage 2 'follow-up sample' of 2,023 AD cases and 2,340 controls. Individuals included in this study were drawn from Europe and the United States. All individuals were of Caucasian ancestry. All AD cases met criteria for either probable (NINCDS-ADRDA, DSM-IV) or definite (CERAD)

AD. This study used 'elderly screened controls' and 'population controls'. Elderly controls were screened for cognitive decline or neuropathological signs of AD.

'Population controls' were drawn from large existing cohorts with available genome-wide association data.

**Stage 1 Discovery Sample:** The discovery sample included 4,113 cases and 1,602 elderly screened controls genotyped at the Sanger Institute on the Illumina 610-quad chip, referred to collectively hereafter as the 610 group. These samples were recruited by the Medical Research Council (MRC) Genetic Resource for AD (Cardiff University; Institute of Psychiatry, London; Cambridge University; Trinity College Dublin), the Alzheimer's Research Trust (ART) Collaboration (University of Nottingham; University of Manchester; University of Southampton; University of Bristol; Queen's University Belfast; the Oxford Project to Investigate Memory and Ageing (OPTIMA), Oxford University); Washington University, St Louis, United States; MRC PRION Unit, University College London; London and the South East Region AD project (LASER-AD), University College London; University of Bonn, Germany and the National Institute of Mental Health (NIMH) AD Genetics Initiative. These data were combined with data from 844 AD cases and 1,255 elderly screened controls ascertained by the Mayo Clinic, Jacksonville, Florida; Mayo Clinic, Rochester, Minnesota; and the Mayo Brain Bank, which were genotyped using the Illumina HumanHap300 BeadChip. These samples were used in a previous GWAS of AD⁴. A total of 6,825 population controls were included in stage 1. These were drawn from large existing cohorts with available GWAS data, including the 1958 British Birth Cohort (1958BC) (http://www.b58cgene.sgul.ac.uk), NINDS funded neurogenetics collection at Coriell Cell Repositories (Coriell) (see http://ccr.coriell.org/), the KORA Study", Heinz Nixdorf Recall Study^{12,13} and ALS Controls. The ALS Controls were genotyped using the Illumina HumanHap300 BeadChip. All other population controls were genotyped using the Illumina HumanHap550 Beadchip. Clinical characteristics of the discovery sample can be found in Table S1.

**Stage 2 Follow-up Sample:** The follow-up sample comprised 2,023 AD cases and 2,340 controls. Samples were drawn from the MRC genetic resource for AD, the ART Collaboration, University of Bonn, Aristotle University of Thessaloniki, a Belgian sample derived from a prospective clinical study at the Memory Clinic and Department of Neurology, ZNA Middelheim, Antwerpen¹⁴ and the University of Munich. Clinical characteristics of the follow-up sample can be found in Table S2.

**Additional Stage 2 Samples;** The additional stage2 sample comprised 1,239 AD cases and 2,724 controls which were used for genotyping of the *ABCA7* and *MS4A* loci. Samples were drawn from the MRC genetic resource for AD, the ART Collaboration, University of Bonn, and Aristotle University of Thessaloniki. Clinical characteristics of the full stage 2 follow-up sample can be found in Table S3.

### DNA extraction and laboratory quality control

DNA was obtained from blood samples from each participant, by phenol/chloroform extraction, followed by precipitation in ethanol and storage in TE buffer (some DNA was extracted using Qiagen kits). Initial DNA concentrations were determined by UV spectrophotometry (µQuant microplate spectrophotometer, Bio-Tek®, Beds, UK) or NanoDrop™ (Thermo Scientific, DE, USA). The concentration of each sample was then determined using the PicoGreen® dsDNA Quantitation Reagent (Molecular Probes®, Eugene, Ore.) in a Labsystems Ascent Fluoroskan® (LifeSciences Int., Basingstoke, UK). Each sample was then diluted to 50ng/ul and allowed to equilibrate at 4°C for 48h. DNA quality was assessed by agarose gel electrophoresis under standard conditions. Samples showing no evidence of degradation were then genotyped in a panel of 30 SNPs using the MassARRAY® and iPlex® systems (Sequenom®, San Diego, CA) following manufacturer's protocols. This allowed gender to be checked and permitted sample identity checks after re-arraying samples for GWAS.

### Stage 1 Genotyping (610 group)

Genotyping was performed at the Sanger Institute, UK. All normalised samples passing quality control (QC) were re-arrayed for GWAS using a Biomek® FX Laboratory Automation Workstation (Beckman Coulter®, Inc., Fullerton, CA) into 96 well plate formats. 200ng of input DNA per sample were used and prepared for genotyping using the Illumina Infinium™ system (Illumina® Inc., San Diego, CA, USA). Manufacturer's protocols were followed throughout. Briefly, DNA was isothermally amplified overnight then enzymatically fragmented, alcohol precipitated and resuspended. In this study we used Illumina Human 610-Quad BeadChips (Illumina® Inc., San Diego, CA, USA) which were prepared for hybridization in a capillary flow-through chamber. The amplified and fragmented DNA samples were hybridised to the bead chips using a Tecan robot and an enzymatic base extension used to confer allele specificity. The chips were subsequently stained and scanned using an iSCAN reader (Illumina® Inc., San Diego, CA, USA) to detect fluorescence at each bead. Data were loaded into Beadstudio and final call reports containing X, Y, X-Raw and Y-Raw outputted. The Illuminus algorithm for cluster analysis was used for genotype calling¹⁵.

### Stage 1: Individual Quality Control

4,113 AD cases and 1,602 controls were genotyped on the Illumina 610-quad chip as part of this study (the 610 group). In addition, 844 AD cases and 8,080 controls previously genotyped using either the Illumina HumanHap550 or Illumina HumanHap300 were included in the analysis. These genotypes were generated as part of 7 different studies, making 8 separate groups in total: 1) 610; 2) Mayo; 3) 1958 birth cohort (Sanger); 4) 1958 birth cohort (T1 DGC); 5) ALS control; 6) Coriell control; 7) Heinz Nixdorf Recall (HNR) study; 8) Kora. As we used genotype data from multiple sources, it was important to apply stringent QC filters, as differential genotyping error rates between groups could result in spurious associations when the data are combined^{16,17}. These filters were applied separately to each of these 8 groups to remove poorly performing samples using tools implemented in PLINK v1.05 (http://pngu.mgh.harvard.edu/∼purcell/plink)¹⁸. The specific QC thresholds applied and the breakdown of samples excluded by group are given in Tables S4 and S5, respectively. We removed 1,469 individuals with missing genotype rates > 0.01. We also applied a filter based on mean autosomal heterozygosity, excluding 578 individuals with values above or below empirically determined thresholds. 71 individuals with inconsistencies between reported gender and genotype-determined gender and 22 individuals with ambiguous genotype-determined gender were removed. All individuals passing these QC filters were examined for potential genetic relatedness by calculating identity by descent (IBD) estimates for all possible pairs of individuals in PLINK, and removing one of each pair with an IBD estimate ≥ 0.125 (the level expected for first cousins). IBD estimates were calculated using SNPs that were common to the Illumina 610, 550 and 300 chips with a genotype missing data rate ≤ 0.01, Hardy-Weinberg *P* ≥ 1x10⁻⁵ and a minor allele frequency ≥ 0.01. As a result, 506 individuals were excluded (note that this includes 311 individuals that were included in both the Coriell and ALS control group).

We also sought to detect non-European ancestry. To this end, genotype data from SNPs typed in all cohorts was merged with genotypes at the same SNPs from 210 unrelated European (CEU), Asian (CHB and JPT) and Yoruban (YRI) samples from the HapMap project. Subsequent to removing SNPs in extensive regions of linkage disequilibrium (chr5:44-51.5 Mb; chr6: 25-33.5 Mb; chr8: 8-12 Mb; char11: 45-57 Mb)¹⁹, we further pruned SNPs if any pair within a 50-SNP window had r² > 0.2. Genome-wide average identity by state (IBS) distance was calculated in PLINK between each pair of individuals in the resulting dataset, based on 57,966 SNPs (all with a genotype missing data rate ≤ 0.01, Hardy-Weinberg *P* ≥ 1x10⁻⁵ and a minor allele frequency ≥ 0.01). The resulting matrix of IBS distances was used as input for classical multi-dimensional scaling (MDS) in R v2.7.1 (http://www.r-project.org). When the first two dimensions were extracted and plotted against each other, three clusters were observed corresponding to the European, Asian and Yoruban samples. Sixteen samples appeared to be ethnic outliers from the European cluster and were excluded from further analysis.

We assessed population structure within the data using principal components analysis (PCA) as implemented in EIGENSTRAT²⁰ to infer continuous axes of genetic variation. Eigenvectors were calculated based on the previously described LD-pruned subset of 57,966 SNPs common to all arrays. The EIGENSTRAT program also identifies genetic outliers, which are defined as individuals whose ancestry is at least 6 standard deviations from the mean on one of the top ten axes of variation. As a result, 188 outliers were identified and excluded. Following sample QC 3,941 AD cases and 7,848 controls were included in the analysis.

### Stage 1: SNP Quality Control

Only autosomal SNPs were included in this analysis. Individuals were genotyped on either the Illumina 610-quad as part of this project, or were previously genotyped on the Illumina HumanHap550 or the Illumina HumanHap300 array, and the genotypes made available to us. Note that SNPs had already been filtered out of some groups prior to inclusion in this study. Moreover, where different versions of the same array were used (*e.g*. HumanHap550v1 used to genotype the 1958 birth cohort (Sanger) cohort compared with the HumanHap550v3 array used to genotype the 1958 birth cohort (T1 DGC)), only SNPs common to both versions were considered as present on that array. As such, SNPs included in our analysis fell into 4 different categories; 1) 266,714 SNPs common to all 3 arrays and genotyped in all individuals; 2) 202,516 SNPs common to the 610 and 550 arrays, but not present or without genotypes in individuals typed on the 300 array; 3) 7,744 SNPs common to the 610 and 300 arrays, but not present or without genotypes in individuals typed on the 550 array; 4) 105,614 SNPs with genotypes only in the 610 data (see Table S6).

We assessed the effects of different missing data rate and Hardy-Weinberg filters, aiming to remove poorly performing SNPs without excluding markers that may show genuine association with AD. For each of the 4 SNP categories, markers were excluded if they had a minor allele frequency (MAF) < 0.01 or a Hardy-Weinberg *P* ≤ 1x10⁻⁵, in either cases or controls. SNPs with a MAF ≥ 0.05 were excluded if they had a genotype missing rate of > 0.03 in either cases or controls; for SNPs with a MAF between 0.01 and 0.05, a more stringent genotype missing rate threshold of 0.01 was employed. As a result of this basic SNP QC 43,542 SNPs were excluded.

Ten principal components (PCs) were extracted using EIGENSTRAT, as previously described. To determine if the PCs could assuage any population structure within our sample, we performed logistic regression tests of association with AD, sequentially including between 0 and 10 of the top PCs as covariates. The impact of including the PCs was evaluated by calculating the genomic control inflation factor, λ²¹. We found that including the first 4 PCs as covariates had the maximum impact on λ (see Table S7).
To minimise inter-chip and inter-cohort differences that could result in an inflation of type I error rate, minor allele frequencies were compared between controls in the different groups using logistic regression analysis, incorporating the top 4 PCs as covariates as previously described. Comparisons were only performed between individuals from the same geographical region (*i.e.* British Isles, Germany or USA) and included: 1) 1958 birth cohort (Sanger) versus 1958 birth cohort (T1 DGC); 2) 1958 birth cohort (combined Sanger and T1 DGC) versus 610 UK controls; 3) 1958 birth cohort (combined Sanger and T1 DGC) versus ALS UK controls; 4) 610 UK controls versus ALS UK controls; 5) HNR cohort versus Kora cohort; 6) 610 German controls versus combined HNR and Kora cohort; 7) Mayo controls versus ALS US controls; 8) Coriell cohort versus combined Mayo and ALS US controls; 9) 610 US controls versus combined Mayo and ALS US controls; 10) 610 US controls versus Coriell cohort. Moreover, as a result of comparisons 2, 4, 6, 7, 9 and 10, elderly screened controls were compared with unscreened/population controls.

For each of the 4 categories of SNPs, a quantile-quantile (Q-Q) plot was produced for each cohort control comparison, and the significance threshold employed to exclude SNPs was based on where the observed χ² statistics departed from the null expectation (see Table S8 and Figure 4). A further 9,828 SNPs were excluded as a result of these comparisons. Thus, a total of 529,218 SNPs were analysed for association with AD in this study.

### Stage 1 Statistical Analysis

SNPs were tested for association with AD using logistic regression, assuming an additive model. Covariates were included in the logistic regression analysis to allow for geographical region and chip, *i.e.* to distinguish between 1) individuals from the British Isles, 2) individuals from Germany, 3) individuals from the US typed on the 610 or 550 chip, 4) individuals from the US typed on the 300 chip. It was not possible to include a covariate for each chip as only controls were genotyped on the 550 chip. Similarly, it was not possible to include a covariate for each of the 8 groups, as only two included both cases and controls (610 and Mayo groups). The first 4 PCs extracted from EIGENSTRAT were also included as covariates, as previously described. Following analysis, 130 cluster plots were visually inspected for SNPs with a p-value ≤ 1x10⁻⁴. Thirteen SNPs showing poorly formed clusters were excluded. Thus our analysis was based on 529,205 SNPs, and a conservative genome-wide significance threshold of 0.05/529205 = 9.4x10⁻⁸ was employed. Q-Q plots of the test results are shown in Figure 5. The overall genomic control inflation factor, λ, was calculated to be 1.037. Results are shown for SNPs with a p-value ≤ 1x10⁻⁴ in Table S9. A breakdown of minor allele frequencies in cases and controls is shown for genome-wide significant SNPs in Table S10.

### Stage 2 Genotyping and Statistical Analysis

We genotyped SNPs in cases and controls from 5 European cohorts (described in Table S2 and S3). Genotyping was performed at Cardiff using the MassARRAY and iPlexGOLD systems (Sequenom, San Diego, CA) according to manufacturer's recommendations. All assays were validated prior to use, based on optimisation in 30 reference Centre d'Etude du Polymorphisme Humain (CEPH) parent - offspring trios. Sample plates contained cases, controls and blank samples. Quality control measures included independent double genotyping blind to sample identity and blind to the other rater, and where available comparison of our CEPH genotypes to those in the HapMap (www.hapmap.org). In addition, 231 individuals included in the GWAS were also genotyped on the Sequenom platform. We calculated the average concordance rate for the 7 SNPs typed on both platforms to be 99%. All genotyped SNPs had genotype call frequency rates >90% in the follow-up sample, and no SNPs had HWE P-value ≤ 0.05 in cases or controls. SNPs were tested for association with AD using logistic regression, assuming an additive model. Covariates were included in the logistic regression analysis to allow for each cohort, i.e. 1) Belgium, 2) MRC, 3) ART, 4) Bonn, 5) Greek.

### CLU and PICALM Meta-analysis

We included genotype data from stages 1 and 2 in a meta-analysis for SNPs at the *CLU* and *PICALM* loci. In addition, we employed genotype data from the TGEN study, a publicly available AD GWAS dataset. This sample is comprised of 861 AD cases and 550 controls genotyped on the Affymetrix 500K chip. If a SNP of interest was not genotyped in our GWAS or the TGEN dataset, an attempt was made to impute genotypes in PLINK, using the 60 HapMap CEU founders as a reference panel. Only imputed SNPs with an information content metric value greater than 0.8 were included in analysis (see PLINK website). SNPs were tested for association with AD using logistic regression, assuming an additive model. Covariates were included in the logistic regression analysis to allow for geographical region and chip as in Stage 1 and for cohort as in Stage 2. Covariates included for the TGEN sample distinguished between samples from the Netherlands Brain Bank and samples from the USA. Results of the meta-analysis are shown in Table 1 and Table S11.

### ABCA7and MS4A Meta-analysis

### Discovery Meta-analysis

We included genotype data from stages 1 and the full stage 2 sample in a meta-analysis for SNPs at the *ABCA7* and *MS4A* loci. In addition, we employed genotype data from the TGEN (Translational Genomics Research Institute)²² and ADNI (Alzheimer's Disease Neuroimaging Initiative)²³ studies, both publicly available AD GWAS datasets, analysed in house for association to AD using a logistic regression assuming an additive model and including country of origin covariates for the TGEN study¹⁰. We first performed an inverse variance weighted fixed effects meta-analysis of the GERAD1, ADNI and TGEN datasets. The *P*-values from this meta-analysis were then combined with the publicly available *P*-values from the EAD11 (European Alzheimer's Disease Initiative Stage 1)²⁴ study using Fisher's combined probability test (note that odds ratios and variances for all SNPs genotyped in the EADI1 study were not publicly available, thus precluding an inverse variance weighted meta-analysis of all 4 GWAS). The combined analysis tested 496763 autosomal SNPs. These SNPs passed QC in our stage 1 sample and each of the ADNI and EADI1 GWA studies; 52391 of these SNPs were also genotyped and passed QC in the TGEN GWAS (which unlike the other studies employed the Affymetrix 500K array). In the combined analysis, 61 SNPs were associated with AD at *P*≤1 x 10⁻⁵ (see Table S14).

### Inverse variance weighted meta-analysis of all available data

For the five SNPs that showed evidence for association in our stage 2 sample (*P*<0.05), summary data (including odds ratios and variances) were obtained from the CHARGE (Cohorts for Heart and Aging Research in Genomic Epidemiology)²⁵ and EADI1 studies, such that all data (*i.e.,* our stage 1 & 2, EADI1, ADNI, TGEN and CHARGE) were combined in an inverse-variance weighted fixed effects meta-analysis (total sample of up to 11607 cases and 31871 controls). Cochran's Q-test was performed and I² calculated to assess heterogeneity. Although the same direction of effect was observed in all studies analyzed for the *CR1* SNP rs3818361, there was some evidence of heterogeneity (Cochran's Q test *P*=0.02, I² = 65%). A random effects meta-analysis of this SNP was also performed for comparison (see Table S15).

### Secondary Analyses

We also tested the GWS SNPs for relationships with age at onset (AAO). To this end, age at onset (in years) was employed as the dependent variable in a linear regression analysis and an additive model was assumed. AAO data was available for 2,856 AD cases. Covariates were included in the logistic regression analysis to allow for geographical region and chip, *i.e.* to distinguish between 1) cases from the British Isles, 2) cases from Germany, 3) cases from the US typed on the 610 chip, 4) cases from the US typed on the 300 chip. Results are shown in Table S12.

In addition, we stratified our sample based on presence/absence of at least 1 *APOE* ε4 allele. We had *APOE* genotype data for 6045 individuals; our ε4-positive sample consisted of 2,203 AD cases and 632 controls; our ε4-negative sample consisted of 1,446 cases and 1,764 controls. We performed genome-wide tests for association with AD in each sub-sample, but no SNP achieved genome-wide significance (data not shown).

### Expected number of significant SNPs

We assessed our results to determine if we observed more significant SNPs at stage 1 than would be expected by chance. We first removed SNPs within 500 kb either side of risk SNPs, *i.e.* rs429358 (the *APOE* ε4 SNP), rs11136000 (*CLU*) and rs3851179 (*PICALM*)*.* We thus excluded 170 *"APOE"* SNPs, 290 *"CLU"* SNPs and 257 *"PICALM"* SNPs. Of the 528,448 remaining SNPs we estimated 397,224.7 "independent" tests using the algorithm we described in (*15*). Of 16 SNPs significant at a significance level α=10⁻⁵ (excluding *APOE, CLU* and *PICALM* SNPs) we estimated 12.6 "independent" tests. We calculated mean (N*α =397224.7*10⁻⁵ ≈ 4.0) and variance (N*α*(1-α) = 3.97) of the expected number of significant tests at α=10⁻⁵ level using the binomial distribution. Thus the probability of observing 12.6 significant tests is P = 7.5x10⁻⁶.

### Linkage Disequilibrium Testing

Preliminary analysis of Novel GWS loci: To further explore relationships within the two associated gene regions *(CLU* and *PICALM*) we selected additional SNPs from each, which either showed some evidence for association at Stage 1 (p≤0.001) or were putative functional SNPs in linkage disequilibrium (D'> 0.3) with the novel GWS hits (see Table S11). Predicted functional SNPs were identified using PupaSuite (http://bioinfo.cipf.es/pupasuite/www/index.jsp)²⁶. These were tested for association with AD in an independent sample and also checked for association in the TGEN GWAS. Four additional SNPs were selected from the *CLU* locus, one which showed some evidence at stage 1 (rs7012010; p = 8x10⁻⁴) and three which had putative functional significance but were not typed in stage 1 (rs3087554, rs9331888 and rs7982, see Table S11). rs7012010 showed no evidence for association in the original Stage 2 sample, some evidence in the TGEN dataset (p = 0.033) but did not reach GWS levels of significance in the meta-analysis (p = 1x10⁻⁴). Two of the remaining SNPs showed no evidence for association, whereas the third rs7982, a synonymous SNP in strong LD (r²=1 in HapMap CEU individuals; r²=0.95 in extension sample) with the GWS SNP in *clusterin,* did show association in the original Stage 2 sample, with a similar magnitude of effect (meta p= 8x10⁻¹⁰; stage 1 genotypes imputed).

We selected 8 additional SNPs for follow-up from the *PICALM* locus: half showed significant evidence for association in Stage 1 and half were chosen for their putative function (see Table S11). Three of the first four SNPs showed evidence for association in both the original Stage 2 and TGEN samples with the fourth showing association in the TGEN dataset alone. Producing combined p-values of: rs677909, p= 3x10⁻⁸; rs541458, p= 5x10⁻⁹; rs543293, p= 1x10⁻⁸; and rs7941541 p = 3x10⁻⁹, none of which exceeded that of the original GWS SNP. All of these SNPs were in high LD with the original GWS SNP. The only potentially functional SNPs which showed good evidence for association were rs561655 which is within a putative transcription factor binding site and rs592297 which is a synonomous SNP in exon 5 of the gene which may influence a putative exon splicing enhancer. However, neither of these SNPs showed the strength of evidence for association observed for rs3851179, the GWS SNP, producing meta p= 1x10⁻⁷ and p= 2x10⁻⁷, respectively.

### Conclusions

Our results provide compelling evidence that the genes described herein are true susceptibility genes for AD. However, a further striking implication of our findings is the support for additional disease mechanisms that go beyond Aβ accumulation. Three, and possibly four of the recently identified AD susceptibility loci (CLU, CR1, ABCA7 & the MS4A gene cluster) have known functions in the immune system, specifically the classical complement pathway. Furthermore, both PICALM and BIN1 are involved in clathrin-mediated endocytosis and APOE, CLU and ABCA7 in lipid processing.

**Table 1. SNPs showing genome-wide significant association with AD in stage 1 of the GWAS. P-values in the original stage 2 extension sample, the combined stage&2 sample, and a meta-analysis of all available data (EADI1, ADNI, TGEN and CHARGE) are also shown for the two SNPs unlinked to the APOE locus (highlighted in bold).**

| SNP | Chr | MB | Closest RefSeq Gene | Location Relative to Gene | MAF | Stage1: 3941 cases 7848 controls | Stage2: 2023 cases 2340 controls | Meta-analysis Stage1&2: 5964 cases 10188 controls | | Meta-analysis Stage 1 &2, EADI1, ADNI, TGEN, CHARGE; 10368 cases, 29147 controls | |
|---|---|---|---|---|---|---|---|---|---|---|---|
| | | | | | | P-value (two-tailed) | P-value (one-tailed) | P-value (two-tailed) | OR (95% CI) | P-value (two-tailed) | OR (95% CI) |
| rs2075650 * | 19 | 50.1 | *TOMM40* | Intron | 0.15 | 1.8x10⁻¹⁵⁷ | | | | | |
| rs157580 | 19 | 50.1 | *TOMM40* | Intron | 0.39 | 9.6x10⁻⁵⁴ | | | | | |
| rs6859 | 19 | 50.1 | *PVRL2* | 3'UTR | 0.43 | 6.9x10⁻⁴¹ | | | | | |
| rs8106922 | 19 | 50.1 | *TOMM40* | Intron | 0.40 | 5.4x10⁻³⁹ | | | | | |
| rs405509 | 19 | 50.1 | *APOE* | 5' | 0.52 | 4.9x10⁻³⁷ | | | | | |
| rs10402271 | 19 | 50.0 | *BCAM* | 3' | 0.32 | 1.5x10⁻²⁶ | | | | | |
| rs439401 † | 19 | 50.1 | *APOE* | 3' | 0.36 | 2.7x10⁻²³ | | | | | |
| rs2927438 | 19 | 49.9 | *BCL3* | 5' | 0.21 | 3.0x10⁻¹¹ | | | | | |
| rs377702 | 19 | 50.1 | *PVRL2* | Intron | 0.38 | 8.4x10⁻¹¹ | | | | | |
| **rs11136000** | **8** | **27.5** | ***CLU*** | **Intron** | **0.40** | **1.4x10⁻⁹** | **0.017** | **8.5x10⁻¹⁰** | **0.86 (0.82-0.90)** | **8.7x10-¹⁹** | **0.86 (0.84-0.89)** |
| rs1048699 | 19 | 50.3 | ***NKPD1*** | 3' | 0.09 | 1.5x10⁻⁹ | | | | | |
| rs1114832 | 19 | 50.3 | *LRRC68* | Intron | 0.09 | 2.2x10⁻⁹ | | | | | |
| rs1871047 | 19 | 50.0 | *PVRL2* | Intron | 0.40 | 1.3x10⁻⁸ | | | | | |
| **rs3851179** | **11** | **85.5** | ***PICALM*** | **5'** | **0.37** | **1.9x10⁻⁸** | **0.014** | **1.3x10⁻⁹** | **0.86 (0.82-0.90)** | **2.4x10⁻¹⁵** | **0.86 (0.84-0.90)** |
| rs5167 | 19 | 50.1 | *APOC2* | Exon | 0.35 | 3.4x10⁻⁸ | | | | | |

| | | | | | | | | | | | |
|---|---|---|---|---|---|---|---|---|---|---|---|
| Chr = Chromosome; MB = position in megabases; MAF = minor allele frequency in controls; OR = odds ratio for the minor allele; 95% CI = 95% confidence interval; UTR= untranslated region. * rs2075650 is in linkage disequilibrium with rs429358, the *APOE* ε4 SNP (r² = 0.48). † This SNP was genotyped in a subsample of 3333 cases and 6460 controls. | | | | | | | | | | | |

**Table 2. SNPs showing association with AD at P≤ 1x10⁻⁵ (excluding SNPs at the APOE, CLU and PICALM loci).**

| SNP | Chr | MB | Closest RefSeq Gene | Location Relative to Gene | GWAS P-value | OR | 95% CI |
|---|---|---|---|---|---|---|---|
| rs11894266 | 2 | 170.3 | *SSB* | 5' | 6.9x10⁻⁷ | 0.86 | 0.81-0.91 |
| rs610932 | 11 | 59.7 | *MS4A6A* | 3'UTR | 1.4x10⁻⁶ | 0.87 | 0.82-0.92 |
| rs10501927 | 11 | 99.3 | *CNTN5* | Intronic | 2.0x10⁻⁶ | 1.18 | 1.10-1.26 |
| rs9446432 | 6 | 72.4 | | Intergenic | 2.8x10⁻⁶ | 1.28 | 1.15-1.41 |
| rs7561528 | 2 | 127.6 | *BIN1* | 5' | 3.0x10⁻⁶ | 1.16 | 1.09-1.24 |
| rs744373 | 2 | 127.6 | *BIN1* | 5' | 3.2x10⁻⁶ | 1.17 | 1.09-1.25 |
| rs662196 | 11 | 59.7 | *MS4A6A* | Intronic | 5.2x10⁻⁶ | 0.88 | 0.83-0.93 |
| rs583791 | 11 | 59.7 | *MS4A6A* | Intronic | 5.3x10⁻⁶ | 0.88 | 0.83-0.93 |
| rs676309 | 11 | 59.8 | *MS4A4E* | 5' | 6.3x10⁻⁶ | 1.14 | 1.08-1.20 |
| rs1157242 | 8 | 37.2 | | Intergenic | 7.0x10⁻⁶ | 1.17 | 1.10-1.26 |
| rs1539053 | 1 | 57.9 | *DAB1* | Intronic | 7.1x10⁻⁶ | 0.88 | 0.83-0.93 |
| rs11827375 | 11 | 76.0 | *C11orf30* | 3' | 7.2x10⁻⁶ | 1.23 | 1.12-1.35 |
| rs1408077 | 1 | 205.9 | *CR1* | Intronic | 8.3x10⁻⁶ | 1.17 | 1.09-1.25 |
| rs9384428 | 6 | 156.5 | | Intergenic | 8.5x10⁻⁶ | 1.14 | 1.08-1.21 |
| rs6701713 | 1 | 205.9 | *CR1* | Intronic | 8.7x10⁻⁶ | 1.17 | 1.09-1.25 |
| rs3818361 | 1 | 205.9 | *CR1* | Intronic | 9.2x10⁻⁶ | 1.17 | 1.09-1.25 |

| | | | | | | | |
|---|---|---|---|---|---|---|---|
| Chr = Chromosome; MB = position in megabases; OR = odds ratio for the minor allele; 95% CI = 95% confidence internal; UTR= untranslated region. | | | | | | | |

**Table 3. Summary statistics for SNPs selected from meta-analysis of stage1, ADNI, TGEN and EADI1 datasets and genotyped in the full stage 2 sample.**

| SNP | Chr | Closet gene | Discovery Stage1, EADI1, ADNI& ADNI & TGEN *P*^{a}: 6978 cases, 13903 controls | Follow-up Stage 2: 3,262 cases, 5064 controls | | Meta-analysis Stage1&2, EADI1, ADNI, TGEN, CHARGE *P*^{b}: 11607 cases, 31871 controls | |
|---|---|---|---|---|---|---|---|
| | | | | *P* | Odds Ratio (95% CI) | *P* | Odds Ratio (95% CI) |
| rs3818361 | 1 | *CR1* | 5.5x10^{-10d} | 8.9x10⁻³ | 1.13 (1.03 - 1.25) | 1.9x10⁻¹² | 1.17(1.12-1.22) |
| rs744373 | 2 | *BIN1* | 2.0x10^{-8d} | 6.5x10⁻³ | 1.12 (1.03 - 1.21) | 2.1x10⁻¹² | 1.14 (1.10 - 1.19) |
| rs10501927 | 11 | *CNTN5* | 1.6x10⁻⁷ | 9.9x10⁻¹ | 1.00 (0.92 - 1.09) | | |
| rs1858973 | 16 | *IQCK* | 4.9x10⁻⁷ | 5.2x10⁻¹ | 0.97 (0.88 - 1.07) | | |
| rs10761558 | 10 | *CDC2* | 1.3x10⁻⁶ | 9.2x10⁻¹ | 1.00 (0.93 - 1.09) | | |
| rs4782279 | 16 | *IQCK* | 1.3x10⁻⁶ | 4.0x10⁻¹ | 0.96 (0.87 - 1.06) | | |
| rs610932 | 11 | *MS4A4E* | 1.5x10^{-6d} | 2.8x10⁻² | 0.92 (0.85 - 0.99) | 1.1x10⁻⁷ | 0.91 (0.88 - 0.94) |
| rs3764650 | 19 | *ABCA7* | 1.5x10^{-6d} | 1.3x10⁻⁴ | 1.27 (1.12 - 1.43) | 7.3x10⁻¹⁰ | 1.22 (1.14 - 1.30) |
| rs4958112 | 5 | *FSTL4* | 1.6x10⁻⁶ | 7.2x10⁻¹ | 1.01 (0.94 - 1.09) | | |
| rs7191155 | 16 | *IQCK* | 2.2x10⁻⁶ | 4.2x10⁻¹ | 0.96 (0.87 - 1.06) | | |
| rs8055533 | 16 | *CLEC16A* | 2.8x10⁻⁶ | 9.5x10⁻¹ | 1.00 (0.93 - 1.07) | | |
| rs739565 | 16 | *IQCK* | 3.3x10⁻⁶ | 7.2x10⁻¹ | 1.01 (0.94 - 1.09) | | |
| rs3135344 | 6 | *BTNL2* | 5.2x10⁻⁶ | 2.3x10⁻¹ | 1.07 (0.96 - 1.19) | | |
| rs3809278 | 12 | *CUX2* | 5.3x10⁻⁶ | 9.1x10⁻¹ | 1.01 (0.90 - 1.13) | | |
| rs670139 | 11 | *MS4A6A* | 5.6x10^{-6d} | 9.2x10⁻⁴ | 1.14 (1.06 - 1.23) | 1.2x10⁻⁹ | 1.12 (1.08 - 1.16) |
| rs11894266 | 2 | *SSB* | 5.9x10⁻⁶ | 0.6821³ | 0.99 (0.92 -1.06) | | |
| rs1539053 | 1 | *DAB1* | 6.7x10⁻⁶ | 2.6x10⁻¹ | 1.05 (0.97 - 1.13) | | |
| rs11767557 | 7 | *EPHA1* | 7.2x10⁻⁶ | 8.4x10⁻¹ | 0.99 (0.90 - 1.09) | | |
| rs9314866 | 9 | *GNAQ* | 8.2x10⁻⁶ | 4.2x10⁻¹ | 0.97 (0.89 - 1.05) | | |
| rs7573507 | 2 | *ARHGAP25* | 8.5x10⁻⁶ | 6.8x10⁻¹ | 0.96 (0.79 - 1.17) | | |
| rs4571225 | 3 | *IL1RAP* | 9.9x10⁻⁶ | 8.1x10⁻¹ | 0.98 (0.79 - 1.20) | | |

| | | | | | | | |
|---|---|---|---|---|---|---|---|
| Chr, chromosome; 95%CI, 95% confidence interval a P values were obtained by performing an inverse-variance weighted meta-analysis of the Stage 1, ADNI and TGEN, and combining the resultant P-values with those from the EADI1 study using Fisher's combined probability test. b Inverse variance weighted meta-analysis c rs11894266 failed to optimize, rs13010581 genotyped as proxy (r2= 1, D'= 1), d P-values based on inverse variance weighted meta-analysis of Stage 1&2, ADNI, TGEN and EADI1 for SNPs rs3764650, rs670139, rs744373, rs610932 & rs3818361were 2.8 x 10-7, 2.9 x 10-6, 3.6 x 10-9, 3.8 x 10-7 and 2.5 x 10-12, respectively. | | | | | | | |

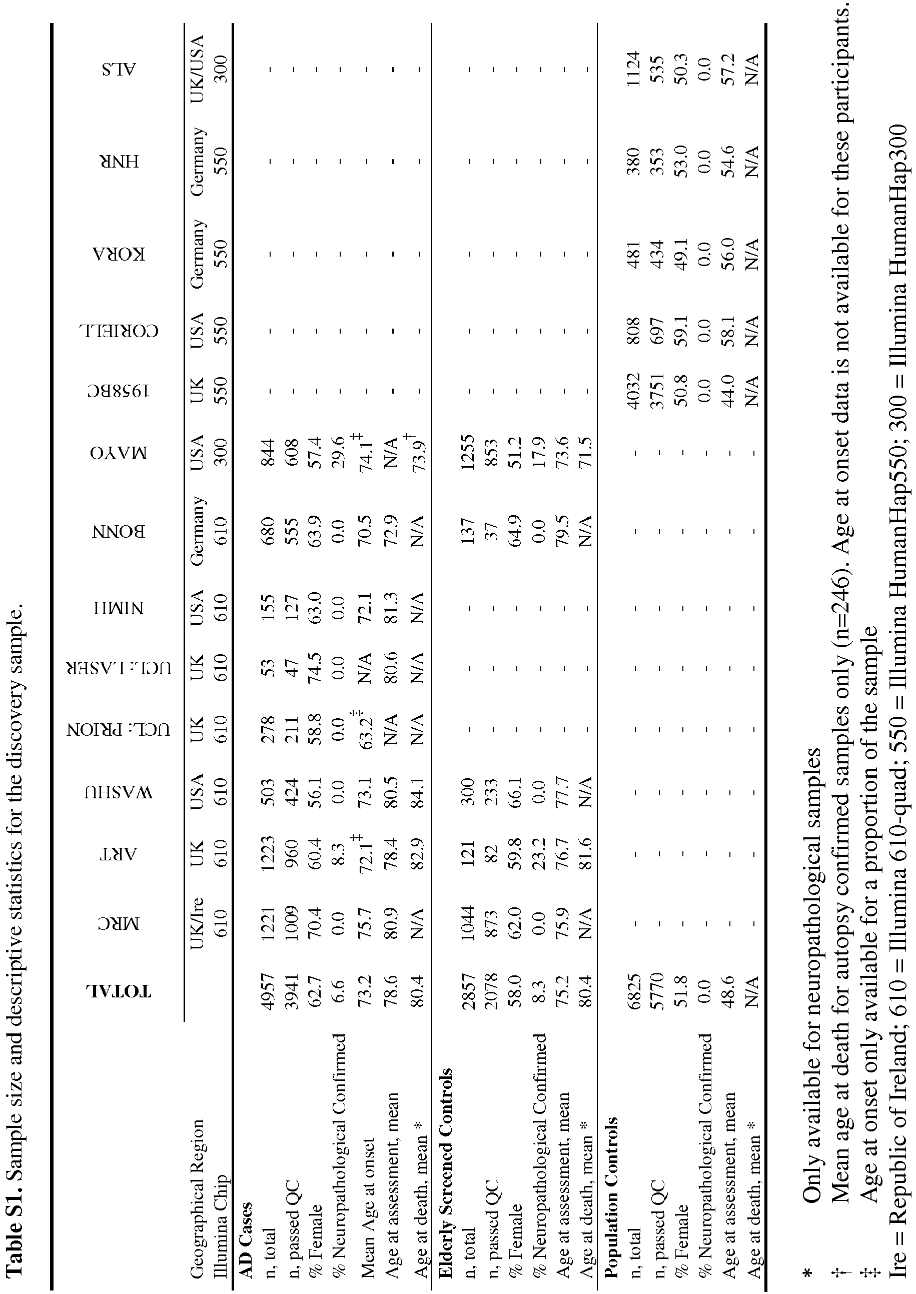

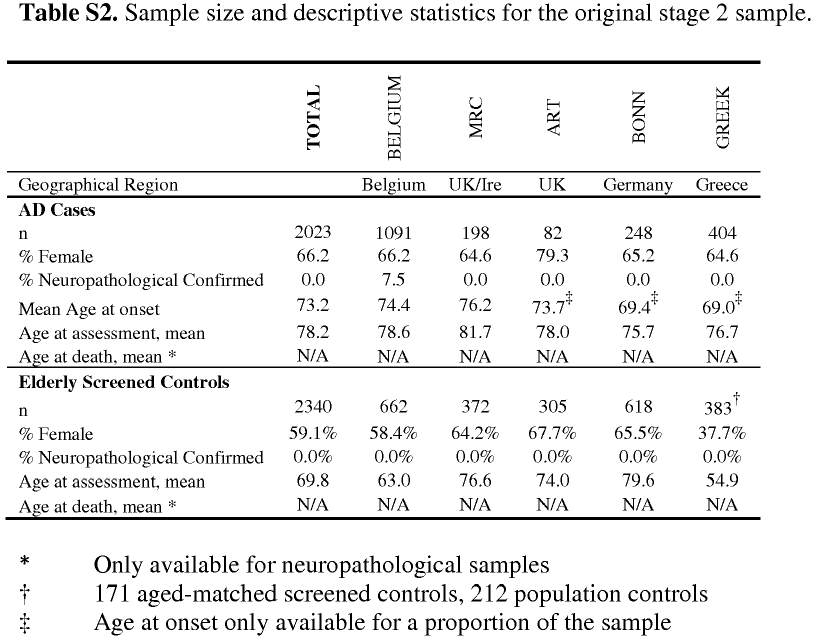

**Table S3. Descriptive statistics for full Stage 2 sample.**

| | Stage2 Sample | | | | | | | | | |
|---|---|---|---|---|---|---|---|---|---|---|
| | Total stage2 Sample | MRC | ART | Belgium | Bonn | Caerphilly | UCL-PRION | Laser | Greece | Munich |
| **Genotyping Platform** | Sequenom | | | | | | | | | |
| **AD Cases** | | | | | | | | | | |
| n | 3262 | 291 | 628 | 1078 | 347 | 52 | 92 | 42 | 404 | 328 |
| % Female | 64.4 | 63.5 | 61.3 | 66.2 | 79.3 | 0 | 57.1 | 69.0 | 64.6 | 66.8 |
| Age at onset, Mean | 72.9 | 75.7 | 70.6‡ | 74.9 | 70.3 | N/A | 61.2 | N/A | 69.0‡ | 70.5 |
| Age at Interview, Mean | 77.7 | 81.1 | 78.4† | 78.6 | 76.2 | N/A | N/A | 79.3 | 76.7 | 73.2 |
| Age at death, Mean | 81.6 | N/A | 81.6† | N/A | N/A | N/A | N/A | N/A | N/A | N/A |
| | | | | | | | | | | |

| **Controls** | | | | | | | | | | |
|---|---|---|---|---|---|---|---|---|---|---|
| n | 5064 | 451 | 399 | 906 | 896 | 0 | 0 | 0 | 364 | 2048 |
| % Female | 56.2 | 62.0 | 60.5 | 58.4 | 68.0 | N/A | N/A | N/A | 37.2 | 51.2 |
| Age at Interview, Mean | 62.4 | 76.5 | 74.0† | 63.0 | 79.6 | N/A | N/A | N/A | 54.2 | 50.7 |
| Age at death, Mean | 76.7 | N/A | 76.7† | N/A | N/A | N/A | N/A | N/A | N/A | N/A |
| | | | | | | | | | | |

| **Incidence studies** | | | | | | | | | | |
|---|---|---|---|---|---|---|---|---|---|---|
| Cohort at risk | | | | | | | | | | |
| % Female | | | | | | | | | | |
| Age at start | | | | | | | | | | |
| Incident AD cases | | | | | | | | | | |

| | | | | | | | | | | |
|---|---|---|---|---|---|---|---|---|---|---|
| † Age at interview not available for 438 AD cases and 104 controls. Age at death is provided for these subjects where available. ‡ Age at onset data only available for less than 75% of the sample | | | | | | | | | | |

**Table S4. Quality control filters applied to individuals in each cohort. N= number of individuals; Chr= chromosome.**

| **Cohort** | **N** | **Illumina Chip** | **Missing Genotype Rate** | **Autosomal Heterozygosity** | **X-Chr Heterozygosity: Males** | **X-Chr Heterozygosity: Females** |
|---|---|---|---|---|---|---|
| 610 | 5715 | 610 | ≤0.01 | 0.325-0.337 | <0.02 | 0.25-0.4 |
| Mayo | 2099 | 300 | ≤0.01 | 0.3455-0.356 | <0.015 | 0.31-0.385 |
| 1958BC (T1DGC) | 2596 | 550 | ≤0.01 | 0.33-0.34 | <0.007 | 0.294-0.37 |
| 1958BC (Sanger) | 1436 | 550 | ≤0.01 | 0.329-0.34 | <0.011 | 0.295-0.38 |
| Coriell | 808 | 550 | ≤0.01 | 0.345-0.356 | <0.015 | 0.32-0.39 |
| ALS Controls | 1124 | 300 | ≤0.01 | 0.345-0.355 | <0.015 | 0.32-0.395 |
| HNR | 380 | 550 | ≤0.01 | 0.33-0.34 | <0.005 | 0.30-0.37 |
| Kora | 481 | 550 | ≤0.01 | 0.33-0.34 | <0.02 | 0.30-0.37 |

**Table S5. Breakdown of individuals excluded by group. Auto Het = mean autosomal heterozygosity. X-Chr Het = mean X chromosome heterozygosity. Note that 311 individuals were included in both the Coriell and ALS control cohort. ^{a} Population controls were not excluded if there was a discrepancy between reported gender and genotype-determined gender. ^{b} Outliers identified by EIGENSTRAT.**

| **Group** | **Number of Individuals before QC** | **Missing Genotype Rate** | **Auto Het** | **X-Chr Het** | **Gender Check ^{a}** | **Relatedness** | **Non-European Ancestry** | **Genetic Outlier^{b}** | **Number of Individuals after QC** |
|---|---|---|---|---|---|---|---|---|---|
| 610 | 5715 | 679 | 140 | 5 | 71 | 168 | 11 | 83 | 4558 |
| Mayo | 2099 | 426 | 151 | 1 | 0 | 19 | 4 | 37 | 1461 |
| 1958BC (T1DGC) | 2596 | 57 | 2 | 2 | 1^{a} | 4 | 0 | 12 | 2519 |
| 1958BC (Sanger) | 1436 | 87 | 108 | 4 | 0 | 1 | 0 | 4 | 1232 |
| Coriell | 808 | 49 | 21 | 8 | 2^{a} | 0 | 1 | 32 | 697 |
| ALS Controls | 1124 | 118 | 139 | 0 | 0 | 313 | 0 | 19 | 535 |
| HNR | 380 | 22 | 4 | 1 | 0 | 0 | 0 | 0 | 353 |
| Kora | 481 | 31 | 13 | 1 | 0 | 1 | 0 | 1 | 434 |
| Total | 14639 | 1469 | 578 | 22 | 71 | 506 | 16 | 188 | 11789 |

**Table S6. Summary of the numbers of SNPs before and after quality control.**

| SNP Category | Chip | Autosomal SNPs before QC | Autosomal SNPs after QC | Cases | Controls |
|---|---|---|---|---|---|
| 1 | 610+550+300 | 266714 | 257253 | 3,941 | 7,848 |
| 2 | 610+550 | 202516 | 199196 | 3,333 | 6,460 |
| 3 | 610+300 | 7744 | 7196 | 3,941 | 2,613 |
| 4 | 610 | 105614 | 65560 | 3,333 | 1,225 |
| | | 582588 | 529205 | | |

**Table S7. The effect of including principal components (PCs) extracted from EIGENSTRAT on the genomic control inflation factor, λ. These values are based on analysis of SNPs common to the Illumina 610-quad, HumanHap550 and HumanHap300 chips (SNP category 1).**

| Principal Components included | λ |
|---|---|
| 0 | 1.079 |
| 1 | 1.047 |
| 1-2 | 1.042 |
| 1-3 | 1.038 |
| 1-4 | 1.038 |
| 1-5 | 1.038 |
| 1-6 | 1.039 |
| 1-7 | 1.038 |
| 1-8 | 1.038 |
| 1-9 | 1.039 |
| 1-10 | 1.040 |

**Table S8. Significance thresholds (χ² values) employed to exclude SNPs showing inter-chip or inter-cohort differences. SNPs with χ² values exceeding these thresholds were excluded.**

| Controls compared | 1 610+550+300 | 2 610+550 | 3 610+300 | 4 610 |
|---|---|---|---|---|
| HNR/Kora | 15.0 | 12.0 | N/A | N/A |
| 610 (German) / HNR+Kora | 17.5 | 10.0 | N/A | N/A |
| 1958BC (Sanger+T1DGC) / ALS (UK) | 6.0 | N/A | N/A | N/A |
| 1958BC (Sanger) / 1958BC (T1DGC) | 11.5 | 10.0 | N/A | N/A |
| 610 (UK) / ALS (UK) | 6.0 | N/A | 13.0 | N/A |
| 1958BC (Sanger+T1DGC) / 610 (UK) | 11.0 | 11.0 | N/A | N/A |
| Mayo / ALS (US) | 10.0 | N/A | 5.0 | N/A |
| Coriell / Mayo+ALS (US) | 25.0 | N/A | N/A | N/A |
| 610 (US) / Mayo+ALS (US) | 25.0 | N/A | 14.0 | N/A |
| 610 (US) / Coriell | 20.0 | 18.5 | N/A | N/A |

| | | | | |
|---|---|---|---|---|
| N/A = not applicable. | | | | |

**Table S9. SNPs showing association with AD (P ≤ 1x10⁻⁴) in the GWAS.**

| SNP | Chr | Position (basepairs) | Minor Allele | Number of Individuals | OR | 95% CI | P-value |
|---|---|---|---|---|---|---|---|
| rs2075650 | 19 | 50087459 | C | 11784 | 2.53 | (2.36-2.71) | 1.8x10⁻¹⁵⁷ |
| rs157580 | 19 | 50087106 | G | 11784 | 0.63 | (0.59-0.66) | 9.6x10-⁵⁴ |
| rs6859 | 19 | 50073874 | T | 11766 | 1.46 | (1.37-1.54) | 6.9x10⁻⁴¹ |
| rs8106922 | 19 | 50093506 | G | 11771 | 0.68 | (0.64-0.71) | 5.4x10⁻³⁹ |
| rs405509 | 19 | 50100676 | C | 11788 | 0.70 | (0.66-0.73) | 4.9x10⁻³⁷ |
| rs10402271 | 19 | 50021054 | G | 11787 | 1.36 | (1.28-1.43) | 1.5x10⁻²⁶ |
| rs439401 | 19 | 50106291 | T | 9773 | 0.72 | (0.67-0.76) | 2.7x10⁻²³ |
| rs2927438 | 19 | 49933947 | T | 11774 | 1.25 | (1.16-1.32) | 3.0x10⁻¹¹ |
| rs377702 | 19 | 50054507 | T | 11779 | 1.20 | (1.13-1.27) | 8.4x10⁻¹¹ |
| rs11136000 | 8 | 27520436 | A | 11756 | 0.84 | (0.79-0.88) | 1.4x10⁻⁹ |
| rs1048699 | 19 | 50342226 | A | 11774 | 1.32 | (1.20-1.43) | 1.5x10⁻⁹ |
| rs1114832 | 19 | 50328041 | A | 11785 | 1.31 | (1.19-1.42) | 2.2x10⁻⁹ |
| rs1871047 | 19 | 50043586 | G | 11784 | 0.85 | (0.80-0.89) | 1.3x10⁻⁸ |
| rs3851179 | 11 | 85546288 | A | 11789 | 0.85 | (0.80-0.89) | 1.9x10⁻⁸ |
| rs5167 | 19 | 50140305 | C | 11789 | 1.17 | (1.10-1.24) | 3.4x10⁻⁸ |
| rs2582367 | 8 | 27535944 | G | 9701 | 0.85 | (0.80-0.90) | 2.1x10⁻⁷ |
| rs7941541 | 11 | 85536186 | C | 9791 | 0.84 | (0.78-0.89) | 2.1x10⁻⁷ |
| rs8103315 | 19 | 49946008 | T | 9788 | 1.24 | (1.14-1.35) | 2.9x10⁻⁷ |
| rs3760627 | 19 | 50149020 | G | 11754 | 1.15 | (1.09-1.21) | 4.0x10⁻⁷ |
| rs2965101 | 19 | 49929652 | C | 11789 | 0.86 | (0.80-0.90) | 4.5x10⁻⁷ |
| rs11894266 | 2 | 170344888 | C | 9793 | 0.86 | (0.80-0.91) | 6.8x10⁻¹ |
| rs543293 | 11 | 85497725 | A | 11787 | 0.86 | (0.81-0.91) | 6.9x10⁻⁷ |
| rs12610605 | 19 | 50062678 | T | 9793 | 0.81 | (0.74-0.88) | 7.7x10⁻⁷ |
| rs1237999 | 11 | 85492678 | C | 11788 | 0.87 | (0.81-0.91) | 1.1x10⁻⁶ |
| rs610932 | 11 | 59695883 | A | 11779 | 0.87 | (0.82-0.92) | 1.4x10⁻⁶ |
| rs10501927 | 11 | 99262939 | G | 11786 | 1.18 | (1.10-1.26) | 2.0x10⁻⁶ |
| rs541458 | 11 | 85465999 | C | 11789 | 0.87 | (0.81-0.91) | 2.3x10⁻⁶ |
| rs9446432 | 6 | 72413481 | C | 9783 | 1.28 | (1.15-1.41) | 2.8x10⁻⁶ |
| rs7561528 | 2 | 127606107 | T | 9779 | 1.16 | (1.09-1.23) | 3.0x10⁻⁶ |
| rs744373 | 2 | 127611085 | G | 9783 | 1.17 | (1.09-1.24) | 3.2x10⁻⁶ |
| rs536841 | 11 | 85465472 | C | 9788 | 0.86 | (0.80-0.91) | 3.5x10⁻⁶ |
| rs659023 | 11 | 85502507 | T | 9759 | 0.86 | (0.81-0.91) | 3.6x10⁻⁶ |
| rs662196 | 11 | 59699333 | G | 11783 | 0.88 | (0.83-0.92) | 5.1x10⁻⁶ |
| rs583791 | 11 | 59703828 | C | 11759 | 0.88 | (0.83-0.92) | 5.3x10⁻⁶ |
| rs440277 | 19 | 50053064 | A | 9793 | 0.86 | (0.80-0.91) | 5.4x10⁻⁶ |
| rs8111069 | 19 | 50175278 | G | 9768 | 1.16 | (1.08-1.23) | 6.1x10⁻⁶ |
| rs676309 | 11 | 59758149 | C | 11785 | 1.14 | (1.07-1.20) | 6.3x10⁻⁶ |
| rs1157242 | 8 | 37158523 | T | 11787 | 1.17 | (1.09-1.25) | 7.0x10⁻⁶ |
| rs1539053 | 1 | 57872295 | T | 11788 | 0.88 | (0.83-0.93) | 7.1x10⁻⁶ |
| rs11827375 | 11 | 75985920 | T | 9775 | 1.23 | (1.12-1.34) | 7.2x10⁻⁶ |
| rs1408077 | 1 | 205870764 | T | 11705 | 1.17 | (1.09-1.25) | 8.3x10⁻⁶ |
| rs9384428 | 6 | 156541223 | G | 11782 | 1.14 | (1.07-1.21) | 8.5x10⁻⁶ |
| rs6701713 | 1 | 205852912 | T | 11786 | 1.17 | (1.09-1.24) | 8.7x10⁻⁶ |
| rs3818361 | 1 | 205851591 | A | 11787 | 1.17 | (1.09-1.24) | 9.2x10⁻⁶ |
| rs1562990 | 11 | 59779663 | C | 11786 | 0.88 | (0.83-0.93) | 1.0x10⁻⁵ |
| rs7933349 | 11 | 99363242 | G | 11785 | 1.15 | (1.07-1.21) | 1.1x10⁻⁵ |
| rs1994313 | 4 | 175953201 | T | 11772 | 0.88 | (0.83-0.93) | 1.2x10⁻⁵ |
| rs618679 | 11 | 85349350 | T | 9775 | 0.84 | (0.78-0.91) | 1.2x10⁻⁵ |
| rs4571225 | 3 | 191807148 | G | 4557 | 0.60 | (0.47-0.75) | 1.3x10⁻⁵ |
| rs569214 | 8 | 27543709 | T | 11788 | 0.88 | (0.83-0.93) | 1.3x10⁻⁵ |
| rs1457850 | 15 | 93332855 | G | 9755 | 0.87 | (0.82-0.92) | 1.4x10⁻⁵ |
| rs3764650 | 19 | 997520 | C | 9790 | 1.25 | (1.12-1.37) | 1.6x10⁻⁵ |
| rs677909 | 11 | 85435237 | G | 11751 | 0.88 | (0.82-0.93) | 1.6x10⁻⁵ |
| rs10761558 | 10 | 62193476 | T | 4549 | 1.26 | (1.13-1.39) | 1.6x10⁻⁵ |
| rs4705563 | 5 | 113007438 | G | 4555 | 0.81 | (0.73-0.89) | 1.7x10⁻⁵ |
| rs10899221 | 11 | 75859279 | T | 9787 | 1.27 | (1.13-1.41) | 1.8x10⁻⁵ |
| rs10425074 | 19 | 50331964 | C | 11778 | 1.15 | (1.07-1.22) | 1.9x10⁻⁵ |
| rs11667640 | 19 | 50071631 | T | 11782 | 0.77 | (0.68-0.87) | 2.3x10⁻⁵ |
| rs667897 | 11 | 59693555 | C | 11777 | 0.89 | (0.84-0.93) | 2.5x10⁻⁵ |
| rs387976 | 19 | 50070900 | C | 9793 | 0.87 | (0.82-0.93) | 2.7x10⁻⁵ |
| rs12781740 | 10 | 64047973 | T | 9789 | 0.83 | (0.76-0.90) | 2.7x10⁻⁵ |
| rs4803750 | 19 | 49939467 | G | 9790 | 0.77 | (0.67-0.86) | 2.8x10⁻⁵ |
| rs272610 | 8 | 81644892 | G | 11781 | 0.89 | (0.83-0.93) | 2.9x10⁻⁵ |
| rs9876068 | 3 | 142901893 | C | 4557 | 0.62 | (0.49-0.77) | 2.9x10⁻⁵ |
| rs7679849 | 4 | 122616456 | A | 11787 | 1.18 | (1.09-1.27) | 3.1x10⁻⁵ |
| rs10898438 | 11 | 85535901 | C | 9780 | 0.88 | (0.83-0.93) | 3.3x10⁻⁵ |
| rs13071397 | 3 | 20805715 | T | 9719 | 1.23 | (1.11-1.36) | 3.8x10⁻⁵ |
| rs11932698 | 4 | 122607113 | A | 11783 | 1.18 | (1.08-1.26) | 3.8x10⁻⁵ |
| rs12449868 | 17 | 69675770 | G | 9784 | 0.88 | (0.82-0.93) | 4.0x10⁻⁵ |
| rs1957325 | 14 | 84965914 | A | 11777 | 0.80 | (0.72-0.89) | 4.0x10⁻⁵ |
| rs4789626 | 17 | 69679180 | T | 11783 | 0.89 | (0.84-0.94) | 4.3x10⁻⁵ |
| rs6716044 | 2 | 104554436 | A | 11784 | 0.81 | (0.73-0.89) | 4.8x10⁻⁵ |
| rs2077815 | 11 | 85350031 | G | 11785 | 0.87 | (0.80-0.92) | 4.9x10⁻⁵ |
| rs7715371 | 5 | 148659187 | T | 9777 | 0.80 | (0.71-0.88) | 5.0x10⁻⁵ |
| rs12692925 | 2 | 170340174 | C | 9791 | 0.88 | (0.82-0.93) | 5.1x10⁻⁵ |
| rs276968 | 16 | 84789677 | G | 11780 | 0.86 | (0.80-0.92) | 5.1x10⁻⁵ |
| rs597668 | 19 | 50400728 | C | 11787 | 1.16 | (1.08-1.24) | 5.2x10⁻⁵ |
| rs2448166 | 8 | 98523312 | G | 11785 | 1.21 | (1.10-1.32) | 5.3x10⁻⁵ |
| rs12686004 | 9 | 106693247 | A | 9778 | 1.21 | (1.10-1.32) | 5.3x10⁻⁵ |
| rs10474519 | 5 | 76929703 | T | 9781 | 1.23 | (1.11-1.35) | 5.4x10⁻⁵ |
| rs1328179 | 1 | 192019846 | T | 11763 | 0.89 | (0.84-0.94) | 5.6x10⁻⁵ |
| rs1474198 | 4 | 72987876 | C | 11785 | 0.89 | (0.84-0.94) | 5.7x10⁻⁵ |
| rs540170 | 11 | 59636614 | T | 9793 | 0.88 | (0.83-0.93) | 5.7x10⁻⁵ |
| rs9539818 | 13 | 63054607 | G | 9763 | 0.81 | (0.72-0.89) | 6.0x10⁻⁵ |
| rs581133 | 11 | 59638882 | C | 9790 | 0.88 | (0.83-0.93) | 6.2x10⁻⁵ |
| rs17005633 | 4 | 83540832 | T | 9761 | 1.18 | (1.08-1.27) | 6.3x10⁻⁵ |
| rs2767576 | 6 | 156921465 | C | 4554 | 1.30 | (1.14-1.48) | 6.3x10⁻⁵ |
| rs527162 | 11 | 85393384 | G | 11789 | 0.87 | (0.80-0.92) | 6.3x10⁻⁵ |
| rs12652626 | 5 | 134511487 | T | 6548 | 1.61 | (1.27-2.02) | 6.4x10⁻⁵ |
| rs10883543 | 10 | 102542742 | C | 11782 | 1.19 | (1.09-1.29) | 6.5x10⁻⁵ |
| rs12671881 | 7 | 153812564 | G | 11775 | 0.89 | (0.84-0.94) | 6.6x10⁻⁵ |
| rs10063333 | 5 | 20623020 | G | 11753 | 1.12 | (1.05-1.18) | 6.6x10⁻⁵ |
| rs17642472 | 5 | 148663280 | T | 9791 | 0.78 | (0.69-0.88) | 6.8x10⁻⁵ |
| rs12697730 | 5 | 82944230 | A | 9792 | 1.14 | (1.06-1.21) | 6.8x10⁻⁵ |
| rs10517459 | 4 | 37636321 | C | 11773 | 1.20 | (1.09-1.3) | 6.8x10⁻⁵ |
| rs4381764 | 2 | 201835108 | C | 4557 | 0.61 | (0.47-0.77) | 6.8x10⁻⁵ |
| rs11606287 | 11 | 47364015 | T | 9677 | 1.13 | (1.06-1.2) | 7.3x10⁻⁵ |
| rs6467136 | 7 | 126952194 | A | 9719 | 1.13 | (1.06-1.19) | 7.3x10⁻⁵ |
| rs1917899 | 11 | 36068192 | C | 9790 | 0.88 | (0.82-0.93) | 7.3x10⁻⁵ |
| rs7255066 | 19 | 49837943 | C | 11778 | 0.88 | (0.82-0.93) | 7.4x10⁻⁵ |
| rs17098701 | 5 | 141836893 | G | 4558 | 0.64 | (0.51-0.79) | 7.4x10⁻⁵ |
| rs309568 | 5 | 82933474 | G | 9792 | 1.14 | (1.06-1.21) | 7.5x10⁻⁵ |
| rs1983659 | 22 | 26252759 | G | 11787 | 0.89 | (0.84-0.94) | 7.7x10⁻⁵ |
| rs7257916 | 19 | 50174724 | G | 9791 | 0.89 | (0.83-0.94) | 7.8x10⁻⁵ |
| rs986251 | 5 | 20606744 | T | 11786 | 1.12 | (1.05-1.18) | 7.9x10⁻⁵ |
| rs7926344 | 11 | 59718742 | A | 9757 | 0.88 | (0.83-0.93) | 7.9x10⁻⁵ |
| rs7017417 | 8 | 27401222 | C | 11787 | 1.18 | (1.08-1.27) | 8.0x10⁻⁵ |
| rs10496105 | 2 | 64174250 | T | 11789 | 1.18 | (1.08-1.28) | 8.1x10⁻⁵ |
| rs1341316 | 1 | 57860377 | C | 11784 | 0.89 | (0.83-0.94) | 8.2x10⁻⁵ |
| rs7929589 | 11 | 59731654 | A | 9791 | 0.88 | (0.83-0.93) | 8.3x10⁻⁵ |
| rs17296283 | 16 | 68143729 | A | 9788 | 1.27 | (1.12-1.42) | 8.5x10⁻⁵ |
| rs6785659 | 3 | 5817729 | A | 9791 | 1.13 | (1.06-1.19) | 8.7x10⁻⁵ |
| rs836326 | 4 | 173387832 | T | 11783 | 1.12 | (1.05-1.18) | 8.8x10⁻⁵ |
| rs4720922 | 7 | 1699628 | T | 11755 | 1.12 | (1.06-1.19) | 9.3x10⁻⁵ |
| rs1241486 | 14 | 24667999 | G | 11775 | 0.90 | (0.84-0.94) | 9.5x10⁻⁵ |
| rs16875079 | 8 | 107860271 | A | 9792 | 1.18 | (1.08-1.28) | 9.5x10⁻⁵ |
| rs12409323 | 1 | 38942258 | T | 4558 | 0.60 | (0.46-0.77) | 9.7x10⁻⁵ |

**Table S10. Breakdown of minor allele frequencies for genome-wide significant SNPs.**

| SNP | Minor allele frequency | | | | | | | | |
|---|---|---|---|---|---|---|---|---|---|
| | UK/Ire cases | UK/Ire screened controls | UK population controls | German cases | German screened controls | German population controls | USA cases | USA screened controls | USA population controls |
| | N=2227 | N=955 | N=3881 | N=555 | N=37 | N=787 | N=1159 | N=1086 | N=1102 |
| rs2075650 | 0.30 | 0.13 | 0.15 | 0.30 | 0.18 | 0.16 | 0.32 | 0.15 | 0.14 |
| rs157580 | 0.30 | 0.42 | 0.38 | 0.27 | 0.38 | 0.35 | 0.26 | 0.39 | 0.38 |
| rs6859 | 0.52 | 0.42 | 0.42 | 0.52 | 0.45 | 0.45 | 0.51 | 0.43 | 0.44 |
| rs8106922 | 0.30 | 0.39 | 0.39 | 0.32 | 0.36 | 0.42 | 0.31 | 0.39 | 0.42 |
| rs405509 | 0.42 | 0.51 | 0.52 | 0.43 | 0.51 | 0.54 | 0.43 | 0.50 | 0.53 |
| rs10402271 | 0.38 | 0.30 | 0.32 | 0.40 | 0.30 | 0.33 | 0.41 | 0.31 | 0.32 |
| rs439401 * | 0.29 | 0.39 | 0.35 | 0.30 | 0.36 | 0.36 | 0.26 | 0.37 | 0.36 |
| rs2927438 | 0.24 | 0.21 | 0.22 | 0.26 | 0.26 | 0.22 | 0.26 | 0.20 | 0.21 |
| rs377702 | 0.42 | 0.35 | 0.38 | 0.43 | 0.42 | 0.41 | 0.44 | 0.38 | 0.41 |
| rs11136000 | 0.37 | 0.40 | 0.40 | 0.35 | 0.41 | 0.41 | 0.36 | 0.39 | 0.40 |
| rs1048699 | 0.12 | 0.09 | 0.09 | 0.11 | 0.14 | 0.09 | 0.11 | 0.08 | 0.09 |
| rs1114832 | 0.12 | 0.09 | 0.10 | 0.12 | 0.14 | 0.09 | 0.12 | 0.09 | 0.09 |
| rs1871047 | 0.37 | 0.41 | 0.40 | 0.35 | 0.39 | 0.37 | 0.34 | 0.40 | 0.39 |
| rs3851179 | 0.34 | 0.38 | 0.38 | 0.34 | 0.38 | 0.36 | 0.33 | 0.36 | 0.36 |
| rs5167 | 0.39 | 0.35 | 0.35 | 0.37 | 0.36 | 0.35 | 0.39 | 0.36 | 0.34 |

| | | | | | | | | | |
|---|---|---|---|---|---|---|---|---|---|
| * This SNP was genotyped in a subsample of 3333 cases and 6460 controls. | | | | | | | | | |

**Table S11. SNPs selected for follow-up genotyping. P-values in the GWAS, the extension sample, a previous AD GWAS (TGEN), and the combined sample (Meta) are also shown. All p-values are two-tailed.**

| SNP | Gene | Reason For Follow Up | LD with GWS SNP | | GWAS P-value (N≤11789) | Extension P-value (N≤4233) | TGEN P-value (N≤1411) | Meta P-value (N<17433) | Meta OR |
|---|---|---|---|---|---|---|---|---|---|
| | | | D' | r² | | | | | |
| rs7982 | *CLU* | Synonymous | 1.000 | 0.312 | 1x10⁻⁹* | 0.032 | N/A | 8x10^{-10†} | 0.86 |
| rs3087554 | *CLU* | 3'UTR | 1.000 | 0.023 | N/A | 0.146 | N/A | 0.146 | 1.09 |
| rs9331888 | *CLU* | 5'UTR (transcript 2) | 1.000 | 0.468 | N/A | 0.304 | N/A | 0.304 | 1.05 |
| rs7012010 | *CLU* | GWAS P<1x10⁻³ | 0.095 | 0.126 | 8x10⁻⁴ | 0.309 | 0.033 * | 1x10⁻⁴† | 1.10 |
| rs561655 | *PICALM* | Within a Putative TFBS | 1.000 | 0.957 | 9x10⁻⁶ * | 0.016 | N/A | 1x10⁻⁷† | 0.87 |
| rs592297 | *PICALM* | Synonymous | 0.199 | 0.708 | 6x10⁻⁵ * | 0.019 | 0.136 * | 2x10⁻⁷† | 0.86 |
| rs636848 | *PICALM* | Within a Putative TFBS | 0.682 | 0.954 | 3x10⁻¹ * | 0.017 | N/A | 2x10⁻²† | 1.07 |
| rs532470 | *PICALM* | Putative eSNP | 0.100 | 0.590 | 7x10⁻² * | 0.498 | N/A | 3x10⁻²† | 1.06 |
| rs7941541 | *PICALM* | GWAS P<1x 10⁻⁴ | 0.960 | 0.875 | 2x10⁻⁷ | 0.189 | 0.005 * | 3x10⁻⁹† | 0.86 |
| rs541458 | *PICALM* | GWAS P<1x 10⁻⁴ | 0.720 | 0.577 | 2x10⁻⁶ | 0.027 | 0.038 | 5x10⁻⁹ | 0.86 |
| rs543293 | *PICALM* | GWAS P<1x 10⁻⁴ | 0.923 | 0.910 | 7x10⁻⁷ | 0.109 | 0.023 | 1x10⁻⁸ | 0.87 |
| rs677909 | *PICALM* | GWAS P<1x 10⁻⁴ | 0.283 | 0.558 | 2x10⁻⁵ | 0.050 | 0.012 | 3x10⁻⁸ | 0.87 |

| | | | | | | | | | |
|---|---|---|---|---|---|---|---|---|---|
| * P-value is based on imputed genotypes. † Meta P-value is based on partially imputed genotypes. GWS= genome-wide significant; OR = odds ratio for the minor allele. | | | | | | | | | |

**Table S12. Test of association between genome-wide significant SNPs and age at onset of Alzheimer's disease.**

| SNP | Chromosome | Position (basepairs) | Number of individuals | β | P-value |
|---|---|---|---|---|---|
| rs2075650 | 19 | 50,087,459 | 2856 | -1.61 | 5.8x10⁻¹² |
| rs157580 | 19 | 50,087,106 | 2856 | 1.41 | 2.5x10⁻⁹ |
| rs6859 | 19 | 50,073,874 | 2854 | -0.80 | 1.8x10⁻⁴ |
| rs8106922 | 19 | 50,093,506 | 2851 | 0.63 | 0.006 |
| rs405509 | 19 | 50,100,676 | 2856 | 0.54 | 0.011 |
| rs10402271 | 19 | 50,021,054 | 2856 | -0.76 | 3.6x10⁻⁴ |
| rs439401 | 19 | 50,106,291 | 2428 | 1.39 | 2.5x10⁻⁷ |
| rs2927438 | 19 | 49,933,947 | 2852 | -0.61 | 0.012 |
| rs377702 | 19 | 50,054,507 | 2856 | -0.58 | 0.007 |
| rs11136000 | 8 | 27,520,436 | 2836 | -0.06 | 0.770 |
| rs1048699 | 19 | 50,342,226 | 2850 | -0.58 | 0.086 |
| rs1114832 | 19 | 50,328,041 | 2855 | -0.48 | 0.146 |
| rs1871047 | 19 | 50,043,586 | 2853 | 0.33 | 0.132 |
| rs3851179 | 11 | 85,546,288 | 2856 | 0.17 | 0.436 |
| rs5167 | 19 | 50,140,305 | 2856 | -0.13 | 0.554 |

**Table S13. P-values for SNPxSNP interaction terms**

| **SNP** | rs744373 (*BIN1*) | rs11136000 (*CLU*) | rs670139 (*MS4A*) | rs3851179 (*PICALM*) | rs3764650 (*ABCA7*) | rs429358 (*APOE*) |
|---|---|---|---|---|---|---|
| rs3818361 (*CR1*) | 0.6607 | 0.4892 | 0.9616 | 0.1942 | 0.9913 | 0.9367 |
| rs744373 (*BIN1*) | | 0.9979 | 0.3080 | 0.9331 | 0.4509 | 0.1270 |
| rs11136000 *(CLU)* | | | 0.2964 | 0.0613 | 0.5545 | 0.6737 |
| rs670139 (*MS4A*) | | | | 0.0502 | 0.3671 | 0.6232 |
| rs3851179 (*PICALM*) | | | | | 0.4491 | 0.7350 |
| rs3764650 (*ABCA7*) | | | | | | 0.6242 |

| | | | | | | |
|---|---|---|---|---|---|---|
| NB: Data calculated from stage1 sample. | | | | | | |

**Table S14. Stage 1 P-values and details of SNP selection for Stage 2 genotyping and Stage 3 meta-anlaysis**

| **SNP** | **CHR** | **POSITION** | **Closest Gene** | **Stage 1 *P*** | **Stage 2 *P*** | **Notes** |
|---|---|---|---|---|---|---|
| rs3764650 | 19 | 997,520 | *ABCA7* | 1.5E-06 | 1.3E-04 | Selected for Stage 3 meta-analysis |
| rs670139 | 11 | 59,728,371 | *MS4A4E* | 5.6E-06 | 9.2E-04 | Selected for Stage 3 meta-analysis |
| rs744373 | 2 | 127,611,085 | *BIN1* | 2.0E-08 | 6.5E-03 | Selected for Stage 3 meta-analysis |
| rs3818361 | 1 | 205,851,591 | *CR1* | 5.5E-12 | 8.9E-03 | Selected for Stage 3 meta-analysis |
| rs610932 | 11 | 59,695,883 | *MS4A6A* | 1.5E-06 | 2.8E-02 | Selected for Stage 3 meta-analysis |
| rs3135344 | 6 | 32,503,014 | *HLA-DRA* | 5.2E-06 | 2.3E-01 | Stage 2 P>0.05, SNP not selected for Stage 3 |
| rs1539053 | 1 | 57,872,295 | *DAB1* | 6.7E-06 | 2.6E-01 | Stage 2 P>0.05, SNP not selected for Stage 3 |
| rs4782279 | 16 | 19,666,508 | *IQCK* | 1.3E-06 | 4.0E-01 | Stage 2 P>0.05, SNP not selected for Stage 3 |
| rs9314866 | 9 | 79,907,769 | *GNAQ* | 8.2E-06 | 4.2E-01 | Stage 2 P>0.05, SNP not selected for Stage 3 |
| rs7191155 | 16 | 19,707,714 | *IQCK* | 2.2E-06 | 4.2E-01 | Stage 2 P>0.05, SNP not selected for Stage 3 |
| rs1858973 | 16 | 19,651,150 | *IQCK* | 4.9E-07 | 5.2E-01 | Stage 2 P>0.05, SNP not selected for Stage 3 |
| rs7573507 | 2 | 68,882,220 | *ARHGAP25* | 8.5E-06 | 6.8E-01 | Stage 2 P>0.05, SNP not selected for Stage 3 |
| rs739565 | 16 | 19,624,006 | *C16orf88* | 3.3E-06 | 7.2E-01 | Stage 2 P>0.05, SNP not selected for Stage 3 |
| rs4958112 | 5 | 132,637,416 | *FSTL4* | 1.6E-06 | 7.2E-01 | Stage 2 P>0.05, SNP not selected for Stage 3 |
| rs4571225 | 3 | 191,807,148 | *IL1RAP* | 9.9E-06 | 8.1E-01 | Stage 2 P>0.05, SNP not selected for Stage 3 |
| rs11767557 | 7 | 142,819,261 | *EPHA1* | 7.2E-06 | 8.4E-01 | Stage 2 P>0.05, SNP not selected for Stage 3 |
| rs3809278 | 12 | 110,209,568 | *CUX2* | 5.3E-06 | 9.1E-01 | Stage 2 P>0.05, SNP not selected for Stage 3 |
| rs10761558 | 10 | 62,193,476 | *CDK1* | 1.2E-06 | 9.2E-01 | Stage 2 P>0.05, SNP not selected for Stage 3 |
| rs8055533 | 16 | 10,949,740 | *CLEC16A* | 2.8E-06 | 9.5E-01 | Stage 2 P>0.05, SNP not selected for Stage 3 |
| rs10501927 | 11 | 99,262,939 | *CNTN5* | 1.6E-07 | 9.9E-01 | Stage 2 P>0.05, SNP not selected for Stage 3 |
| | | | | | | SNP assay failed optimisation; rs13010581, a proxy SNP (r²=1) was genotyped in |
| rs11894266 | 2 | 170,344,888 | *SSB* | 5.9E-06 | N/A | GERAD2 (P=0.682) |
| | | | | | | SNP was not selected for Stage 2 as a proxy SNP rs3818361 (r²=0.956) was genotyped |
| rs1408077 | 1 | 205,870,764 | *CR1* | 7.0E-11 | N/A | through GERAD2 (P=0.009) |
| | | | | | | SNP was not selected for Stage 2 as a proxy SNP rs3818361 (r²=1) was genotyped |
| rs6701713 | 1 | 205,852,912 | *CR1* | 5.2E-11 | N/A | through GERAD2 (P=0.009) |
| | | | | | | SNP was not selected for Stage 2 as a proxy SNP rs610932 (r²=0.88) was genotyped |
| rs667897 | 11 | 59,693,555 | *MS4A6A* | 3.0E-06 | N/A | through GERAD2 (P=0.028) |
| | | | | | | SNP was not selected for Stage 2 as it is in LD with rs610932 (r²=0.75) which was genotyped |
| rs2847666 | 11 | 59,616,152 | *MS4A2* | 4.4E-06 | N/A | through GERAD2 (P=0.028) |
| | | | | | | SNP was not selected for Stage 2 as a proxy SNP rs670139 (r²=1) was genotyped |
| rs676309 | 11 | 59,758,149 | *MS4A4E* | 6.3E-07 | N/A | through GERAD2 (P=0.0009) |
| rs405509 | 19 | 50,100,676 | *APOE* | 1.0E-16 | N/A | SNP was not selected for Stage 2 as it is located at the *APOE* locus |
| rs3760627 | 19 | 50,149,020 | *CLPTM1* | 1.1E-08 | N/A | SNP was not selected for Stage 2 as it is located at the *APOE* locus |
| rs5167 | 19 | 50,140,305 | *APOC4* | 5.7E-09 | N/A | SNP was not selected for Stage 2 as it is located at the *APOE* locus |
| rs1871045 | 19 | 50,018,608 | *BCAM* | 5.6E-06 | N/A | SNP was not selected for Stage 2 as it is located at the *APOE* locus |
| rs10402271 | 19 | 50,021,054 | *BCAM* | 1.0E-16 | N/A | SNP was not selected for Stage 2 as it is located at the *APOE* locus |
| rs8103315 | 19 | 49,946,008 | *BCL3* | 6.4E-12 | N/A | SNP was not selected for Stage 2 as it is located at the *APOE* locus |
| rs2927438 | 19 | 49,933,947 | *BCL3* | 1.5E-14 | N/A | SNP was not selected for Stage 2 as it is located at the *APOE* locus |
| rs4803750 | 19 | 49,939,467 | *BCL3* | 2.7E-07 | N/A | SNP was not selected for Stage 2 as it is located at the *APOE* locus |
| rs2927488 | 19 | 49,923,318 | *CEACAM16* | 6.4E-07 | N/A | SNP was not selected for Stage 2 as it is located at the *APOE* locus |
| rs2965101 | 19 | 49,929,652 | *BCL3* | 4.8E-14 | N/A | SNP was not selected for Stage 2 as it is located at the *APOE* locus |
| rs7257916 | 19 | 50,174,724 | *CLPTM1* | 6.8E-06 | N/A | SNP was not selected for Stage 2 as it is located at the *APOE* locus |
| rs8111069 | 19 | 50,175,278 | *CLPTM1* | 2.7E-07 | N/A | SNP was not selected for Stage 2 as it is located at the *APOE* locus |
| rs2627641 | 19 | 50,400,598 | *EXOC3L2* | 3.9E-06 | N/A | SNP was not selected for Stage 2 as it is located at the *APOE* locus |
| rs597668 | 19 | 50,400,728 | *EXOC3L2* | 5.6E-07 | N/A | SNP was not selected for Stage 2 as it is located at the *APOE* locus |
| rs439401 | 19 | 50,106,291 | *APOE* | 1.0E-16 | N/A | SNP was not selected for Stage 2 as it is located at the *APOE* locus |
| rs1114832 | 19 | 50,328,041 | *LRRC68* | 2.2E-11 | N/A | SNP was not selected for Stage 2 as it is located at the *APOE* locus |
| rs1048699 | 19 | 50,342,226 | *LRRC68* | 4.2E-10 | N/A | SNP was not selected for Stage 2 as it is located at the *APOE* locus |
| rs7255066 | 19 | 49,837,943 | *PVR* | 9.8E-07 | N/A | SNP was not selected for Stage 2 as it is located at the *APOE* locus |
| rs1871047 | 19 | 50,043,586 | *PVRL2* | 9.6E-13 | N/A | SNP was not selected for Stage 2 as it is located at the *APOE* locus |
| rs377702 | 19 | 50,054,507 | *PVRL2* | 3.2E-11 | N/A | SNP was not selected for Stage 2 as it is located at the *APOE* locus |
| rs12610605 | 19 | 50,062,678 | *PVRL2* | 1.8E-09 | N/A | SNP was not selected for Stage 2 as it is located at the *APOE* locus |
| rs11667640 | 19 | 50,071,631 | *PVRL2* | 3.4E-06 | N/A | SNP was not selected for Stage 2 as it is located at the *APOE* locus |
| rs6859 | 19 | 50,073,874 | *PVRL2* | 1.0E-16 | N/A | SNP was not selected for Stage 2 as it is located at the *APOE* locus |
| rs157580 | 19 | 50,087,106 | *TOMM40* | 1.0E-16 | N/A | SNP was not selected for Stage 2 as it is located at the *APOE* locus |
| rs2075650 | 19 | 50,087,459 | *TOMM40* | 1.0E-16 | N/A | SNP was not selected for Stage 2 as it is located at the *APOE* locus |
| rs8106922 | 19 | 50,093,506 | *TOMM40* | 1.0E-16 | N/A | SNP was not selected for Stage 2 as it is located at the *APOE* locus |
| rs11136000 | 8 | 27,520,436 | *CLU* | 4.4E-16 | N/A | SNP was not selected for Stage 2 as it is located at the *CLU* locus |
| rs1237999 | 11 | 85,492,678 | *PICALM* | 3.6E-06 | N/A | SNP was not selected for Stage 2 as it is located at the *PICALM* locus |
| rs543293 | 11 | 85,497,725 | *PICALM* | 9.4E-08 | N/A | SNP was not selected for Stage 2 as it is located at the *PICALM* locus |
| rs659023 | 11 | 85,502,507 | *PICALM* | 4.5E-07 | N/A | SNP was not selected for Stage 2 as it is located at the *PICALM* locus |
| rs7941541 | 11 | 85,536,186 | *PICALM* | 9.1E-08 | N/A | SNP was not selected for Stage 2 as it is located at the *PICALM* locus |
| rs3851179 | 11 | 85,546,288 | *PICALM* | 7.5E-09 | N/A | SNP was not selected for Stage 2 as it is located at the *PICALM* locus |
| rs677909 | 11 | 85,435,237 | *PICALM* | 8.7E-08 | N/A | SNP was not selected for Stage 2 as it is located at the *PICALM* locus |
| rs536841 | 11 | 85,465,472 | *PICALM* | 1.7E-08 | N/A | SNP was not selected for Stage 2 as it is located at the *PICALM* locus |
| rs541458 | 11 | 85,465,999 | *PICALM* | 2.9E-08 | N/A | SNP was not selected for Stage 2 as it is located at the *PICALM* locus |

**Table S15. Results for the 5 SNPs analysed in Stage 3 meta-analysis**

| **Dataset** | **SNP** | **CHR** | **BP** | **Closest gene** | **A1** | **A2** | **OR** | **Lower 95% CI** | **Upper 95% CI** | ***P*** | **Cochran's Q test P** | **I²** |
|---|---|---|---|---|---|---|---|---|---|---|---|---|
| GERAD1 | rs3764650 | 19 | 997,520 | *ABCA7* | G | T | 1.25 | 1.13 | 1.38 | 1.6E-05 | | |
| ADNI | rs3764650 | 19 | 997,520 | *ABCA7* | G | T | 1.01 | 0.58 | 1.75 | 9.7E-01 | | |
| TGEN | rs3764650 | 19 | 997,520 | *ABCA7* | N/A | N/A | N/A | N/A | N/A | N/A | | |
| EADI1 | rs3764650 | 19 | 997,520 | *ABCA7* | G | T | 1.21 | 1.08 | 1.37 | 4.0E-03 | | |
| GERAD2 | rs3764650 | 19 | 997,520 | *ABCA7* | G | T | 1.27 | 1.12 | 1.43 | 1.3E-04 | | |
| CHARGE | rs3764650 | 19 | 997,520 | *ABCA7* | G | T | 1.02 | 0.83 | 1.26 | 8.6E-01 | | |
| **Meta-analysis (Fixed effects)** | **rs3764650** | **19** | **997,520** | ***ABCA7*** | **G** | **T** | **1.22** | **1.14** | **1.30** | **7.3E-10** | **0.43** | **0%** |

| **Dataset** | **SNP** | **CHR** | **BP** | **Closest gene** | **A1** | **A2** | **OR** | **Lower 95% CI** | **Upper 95% CI** | ***P*** | **Cochran's Q test P** | **I²** |
|---|---|---|---|---|---|---|---|---|---|---|---|---|
| GERAD1 | rs670139 | 11 | 59,728,371 | *MS4A4E* | T | G | 1.13 | 1.06 | 1.20 | 1.0E-04 | | |
| ADNI | rs670139 | 11 | 59,728,371 | *MS4A4E* | T | G | 1.08 | 0.78 | 1.49 | 6.6E-01 | | |
| TGEN | rs670139 | 11 | 59,728,371 | *MS4A4E* | T | G | 1.27 | 1.08 | 1.49 | 3.0E-03 | | |
| EADI1 | rs670139 | 11 | 59,728,371 | *MS4A4E* | T | G | 1.06 | 0.98 | 1.14 | 1.2E-01 | | |
| GERAD2 | rs670139 | 11 | 59,728,371 | *MS4A4E* | T | G | 1.14 | 1.06 | 1.23 | 9.2E-04 | | |
| CHARGE_nonimputed | rs670139 | 11 | 59,728,371 | *MS4A4E* | T | G | 1.12 | 1.01 | 1.24 | 3.6E-02 | | |
| **Meta-analysis (Fixed effects)** | **rs670139** | **11** | **59,728,371** | ***MS4A4E*** | **T** | **G** | **1.12** | **1.08** | **1.16** | **1.2E-09** | **0.43** | **0%** |

| **Dataset** | **SNP** | **CHR** | **BP** | **Closest gene** | **A1** | **A2** | **OR** | **Lower 95% CI** | **Upper 95% CI** | ***P*** | **Cochran's Q test *P*** | **I²** |
|---|---|---|---|---|---|---|---|---|---|---|---|---|
| GERAD1 | rs610932 | 11 | 59,695,883 | *MS4A6A* | T | G | 0.87 | 0.82 | 0.92 | 1.4E-06 | | |
| ADNI | rs610932 | 11 | 59,695,883 | *MS4A6A* | T | G | 0.88 | 0.64 | 1.22 | 4.5E-01 | | |
| TGEN | rs610932 | 11 | 59,695,883 | *MS4A6A* | N/A | N/A | N/A | N/A | N/A | N/A | | |
| EADI1 | rs610932 | 11 | 59,695,883 | *MS4A6A* | T | G | 0.93 | 0.86 | 1.00 | 4.6E-02 | | |
| GERAD2 | rs610932 | 11 | 59,695,883 | *MS4A6A* | T | G | 0.92 | 0.85 | 0.99 | 2.8E-02 | | |
| CHARGE_nonimputed | rs610932 | 11 | 59,695,883 | *MS4A6A* | T | G | 0.97 | 0.89 | 1.06 | 4.8E-01 | | |
| **Meta-analysis (Fixed effects)** | **rs610932** | **11** | **59,695,883** | ***MS4A6A*** | **T** | **G** | **0.91** | **0.88** | **0.94** | **1.1E-07** | **0.30** | **18%** |

| **Dataset** | **SNP** | **CHR** | **BP** | **Closest gene** | **A1** | **A2** | **OR** | **Lower 95% CI** | **Upper 95% CI** | ***P*** | **Cochran's Q test *P*** | **I²** |
|---|---|---|---|---|---|---|---|---|---|---|---|---|
| GERAD1 | rs744373 | 2 | 127,611,085 | *BIN1* | G | A | 1.17 | 1.09 | 1.25 | 3.2E-06 | | |
| ADNI | rs744373 | 2 | 127,611,085 | *BIN1* | G | A | 1.13 | 0.83 | 1.56 | 4.4E-01 | | |
| TGEN | rs744373 | 2 | 127,611,085 | *BIN1* | N/A | N/A | N/A | N/A | N/A | N/A | | |
| EADI1 | rs744373 | 2 | 127,611,085 | *BIN1* | G | A | 1.15 | 1.06 | 1.25 | 4.0E-04 | | |
| GERAD2 | rs744373 | 2 | 127,611,085 | *BIN1* | G | A | 1.12 | 1.03 | 1.21 | 6.5E-03 | | |
| CHARGE_nonimputed | rs744373 | 2 | 127,611,085 | *BIN1* | G | A | 1.12 | 1.04 | 1.21 | 4.6E-03 | | |
| **Meta-analysis (Fixed effects)** | **rs744373** | **2** | **127,611,085** | ***BIN1*** | **G** | **A** | **1.14** | **1.10** | **1.19** | **2.1E-12** | **0.92** | **0%** |

| **Dataset** | **SNP** | **CHR** | **BP** | **Closest gene** | **A1** | **A2** | **OR** | **Lower 95% CI** | **Upper 95% CI** | ***P*** | **Cochran's Q test *P*** | **I²** |
|---|---|---|---|---|---|---|---|---|---|---|---|---|
| GERAD1 | rs3818361 | 1 | 205,851,591 | *CR1* | A | G | 1.17 | 1.09 | 1.25 | 9.2E-06 | | |
| ADNI | rs3818361 | 1 | 205,851,591 | *CR1* | A | G | 1.58 | 1.06 | 2.35 | 2.4E-02 | | |
| TGEN | rs3818361 | 1 | 205,851,591 | *CR1* | N/A | N/A | N/A | N/A | N/A | N/A | | |
| EADI1 | rs3818361 | 1 | 205,851,591 | *CR1* | A | G | 1.28 | 1.17 | 1.40 | 8.5E-08 | | |
| GERAD2 | rs3818361 | 1 | 205,851,591 | *CR1* | A | G | 1.13 | 1.03 | 1.25 | 8.9E-03 | | |
| CHARGE | rs3818361 | 1 | 205,851,591 | *CR1* | A | G | 1.04 | 0.94 | 1.16 | 4.7E-01 | | |
| **Meta-analysis (Fixed effects)** | **rs3818361** | **1** | **205,851,591** | ***CR1*** | **A** | **G** | **1.17** | **1.12** | **1.22** | **1.9E-12** | **0.02** | **64%** |
| **Meta-analysis (Random effects)** | **rs3818361** | **1** | **205,851,591** | ***CR1*** | **A** | **G** | **1.17** | **1.08** | **1.26** | **1.2E-04** | | |

**Table S16. Flanking sequence (± 20 bases) of each SNP of interest.**

| **SNP** | **Closest Gene** | **Flanking Sequence** |
|---|---|---|
| rs11136000 | *CLU* | agccacaccagctatcaaaa[T/C]tctctaacgggcccttgcca |
| rs7982 | *CLU* | gccatggacatccacttcca[T/C]agcccggccttccagcaccc |
| rs3851179 | *PICALM* | gttatgtgtgaagtcattta[T/C]aatagatagtgttgataata |
| rs561655 | *PICALM* | gttaacctgggagtgaacta[G/A]acattaaaggggcagcatac |
| rs592297 | *PICALM* | aaatcaagaagtgcatccat[C/T]tgattctgaataattggtac |
| rs1408077 | *CRI* | tctcagtagtggggttttgt[C/A]acctttactgttattattag |
| rs6701713 | *CRI* | tgcctgcagcccaacagatg[G/A]cagtgtgcttaacagctctg |
| rs3818361 | *CRI* | tataccccgttaaaggaaac[G/A]atatagaatacgaatggtct |
| rs7561528 | *BIN1* | tagtttcaagtaaacatgtc[G/A]cagtgaagtttgttgtagag |
| rs744373 | *BIN1* | ccctgtccgtccagactccg[A/G]gagtctccgacgggtactac |
| rs3764650 | *ABCA7* | caggctgcgaactttgcacc[T/G]ttacaccactccacgtgacc |
| rs1562990 | *MS4A4A* | caccacacacaaggcctgaa[C/A]gatcaaaggctgaagagatg |
| rs667897 | *MS4A6A* | cgctccaaacccgctgtgtc[A/G]taccataccggatgtttacc |
| rs676309 | *MS4A4E* | aatggtgagataggtataga[A/G]tagactgtctcagttcaaaa |
| rs583791 | *MS4A6A* | ggcaaagaggggaggaagat[G/A]ccaatagcttagattccca |
| rs662196 | *MS4A6A* | gtttgggcatctgggggaaa[G/A]ccaggtttatgtaaatcaaa |
| rs610932 | *MS4A6A* | agtctgaatttccagaaaac[A/C]atgatcattcaatggatcac |
| rs670139 | *MS4A4E* | atctccaagtcaaagtttac[C/A]tcaagttgggccaatccctg |

| | | |
|---|---|---|
| NB: The position of the SNP of interest within the flanking sequence is depicted by the square brackets. The nucleotide change at this SNP is displayed within the square brackets. | | |

### References

1. Abraham, R. A genome-wide association study for late-onset Alzheimer's disease using DNA pooling. BMC Med. Genomics 1, 44 (2008).
2. Beecham, G. Genome-wide association study implicates a chromosome 12 risk locus for late-onset Alzheimer disease. Am. J. Hum. Genet. 84, 35-43 (2009).
3. Bertram, L. Genome-wide association analysis reveals putative Alzheimer's disease susceptibility loci in addition to APOE. Am. J. Hum. Genet. 83, 623-632 (2008).
4. Carrasquillo, M. Genetic variation in PCDH11X is associated with susceptibility to late-onset Alzheimer's disease. Nat. Genet. 41, 192-198 (2009).
5. Coon, K. A high-density whole-genome association study reveals that APOE is the major susceptibility gene for sporadic late-onset Alzheimer's disease. J. Clin. Psychiatry 68, 613-618 (2007).
6. Grupe, A. Evidence for novel susceptibility genes for late-onset Alzheimer's disease from a genome-wide association study of putative functional variants. Hum. Mol. Genet. 16, 865-873 (2007).
7. Li, H. Candidate single-nucleotide polymorphisms from a genomewide association study of Alzheimer disease. Arch. Neurol. 65, 45-53 (2008).
8. Reiman, E. GAB2 alleles modify Alzheimer's risk in APOE epsilon4 carriers. Neutron 54, 713-720 (2007).
9. Harold, D. et al. Genome-wide association study identifies variants at CLU and PICALM associated with Alzheimer's disease. Nat Genet 41, 1088-1093 (2009).
10. Hollingworth, P. et al. Evidence that ABCA7 and MS4A are novel susceptibility loci for Alzheimer's disease and further support for BIN1 and CR1. Nature Genetics Submitted**,**
11. Wichmann, H., Gieger, C. & Illig, T. KORA-gen--resource for population genetics, controls and a broad spectrum of disease phenotypes. Gesundheitswesen 67 Suppl 1, S26-30 (2005).
12. Birnbaum, S. et al. Key susceptibility locus for nonsyndromic cleft lip with or without cleft palate on chromosome 8q24. Nat. Genet 41, 473-477 (2009).
13. Hillmer, A.M. et al. Susceptibility variants for male-pattern baldness on chromosome 20p11. Nat. Genet 40, 1279-1281 (2008).
14. Brouwers, N. et al. Genetic variability in progranulin contributes to risk for clinically diagnosed Alzheimer disease. Neurology 71, 656-664 (2008).
15. Teo, Y.Y. et al. A genotype calling algorithm for the Illumina BeadArray platform. Bioinformatics 23, 2741-2746 (2007).
16. Clayton, D.G. et al. Population structure, differential bias and genomic control in a large-scale, case-control association study. Nat Genet 37, 1243-1246 (2005).
17. Moskvina, V., Craddock, N., Holmans, P., Owen, M.J. & O’Donovan, M.C. Effects of Differential Genotyping Error Rate on the Type I Error Probability of Case-Control Studies. Hum Hered 61, 55-64 (2006).
18. Purcell, S. et al. PLINK: A Tool Set for Whole-Genome Association and Population-Based Linkage Analyses. The American Journal of Human Genetics 81, 559-575 (2007).
19. Price, A.L. et al. Long-Range LD Can Confound Genome Scans in Admixed Populations. The American Journal of Human Genetics 83, 132-135 (2008).
20. Price, A.L. et al. Principal components analysis corrects for stratification in genome-wide association studies. Nat. Genet 38, 904-909 (2006).
21. Devlin, B. & Roeder, K. Genomic control for association studies. Biometrics 55, 997-1004 (1999).
22. Reiman, E.M. et al. GAB2 alleles modify Alzheimer's risk in APOE epsilon4 carriers. Neuron 54, 713-720 (2007).
23. Potkin, S.G. et al. Hippocampal atrophy as a quantitative trait in a genome-wide association study identifying novel susceptibility genes for Alzheimer's disease. PLoS ONE 4, e6501 (2009).
24. Lambert, J. et al. Genome-wide association study identifies variants at CLU and CR1 associated with Alzheimer's disease. Nat. Genet 41, 1094-1099 (2009).
25. Seshadri, S. et al. Genome-wide analysis of genetic loci associated with Alzheimer disease. JAMA 303, 1832-1840 (2010).
26. Conde, L. et al. PupaSuite: finding functional single nucleotide polymorphisms for large-scale genotyping purposes. Nucleic Acids Res 34, W621-625 (2006).

### SEQUENCE LISTING

<110> University College Cardiff Consultants Limited
OWEN, Michael J
WILLIAMS, Julie
<120> Diagnosis of Alzheimer's Disease
<130> UC3.67
<140> GB 0917326.1
   <141> 2009-10-03
<150> GB0911539.5
   < 151 > 2009-07-03
<150> GB0913357.0
   <151> 2009-07-31
<160> 18
<170> Patentln version 3.1
<210> 1
   <211> 41
   <212> DNA
   <213> Homo Sapiens
<400> 1
   agccacacca gctatcaaaa ttctctaacg ggcccttgcc a 41
<210> 2
   <211> 41
   <212> DNA
   <213> Homo Sapiens
<400> 2
   gccatggaca tccacttcca tagcccggcc ttccagcacc c 41
<210> 3
   <211> 41
   <212> DNA
   <213> Homo Sapiens
<400> 3
   gttatgtgtg aagtcattta taatagatag tgttgataat a 41
<210> 4
   <211> 41
   <212> DNA
   <213> Homo Sapiens
<400> 4
   gttaacctgg gagtgaacta gacattaaag gggcagcata c 41
<210> 5
   <211> 41
   <212> DNA
   <213> Homo Sapiens
<400> 5
   aaatcaagaa gtgcatccat ctgattctga ataattggta c 41
<210> 6
   <211> 41
   <212> DNA
   <213> Homo Sapiens
<400> 6
   tctcagtagt ggggttttgt cacctttact gttattatta g 41
<210> 7
   <211> 41
   <212> DNA
   <213> Homo Sapiens
<400> 7
   tgcctgcagc ccaacagatg gcagtgtgct taacagctct g 41
<210> 8
   <211> 41
   <212> DNA
   <213> Homo Sapiens
<400> 8
   tataccccgt taaaggaaac gatatagaat acgaatggtc t 41
<210> 9
   <211> 41
   <212> DNA
   <213> Homo Sapiens
<400> 9
   tagtttcaag taaacatgtc gcagtgaagt ttgttgtaga g 41
<210> 10
   <211> 41
   <212> DNA
   <213> Homo Sapiens
<400> 10
   ccctgtccgt ccagactccg agagtctccg acgggtacta c 41
<210> 11
   <211> 41
   <212> DNA
   <213> Homo Sapiens
<400> 11
   caggctgcga actttgcacc tttacaccac tccacgtgac c 41
<210> 12
   <211> 41
   <212> DNA
   <213> Homo Sapiens
<400> 12
   caccacacac aaggcctgaa cgatcaaagg ctgaagagat g 41
<210> 13
   <211> 41
   <212> DNA
   <213> Homo Sapiens
<400> 13
   cgctccaaac ccgctgtgtc ataccatacc ggatgtttac c 41
<210> 14
   <211> 41
   <212> DNA
   <213> Homo Sapiens
<400> 14
   aatggtgaga taggtataga atagactgtc tcagttcaaa a 41
<210> 15
   <211> 40
   <212> DNA
   <213> Homo Sapiens
<400> 15
   ggcaaagagg ggaggaagat gccaatagct tagattccca 40
<210> 16
   <211> 41
   <212> DNA
   <213> Homo Sapiens
<400> 16
   gtttgggcat ctgggggaaa gccaggttta tgtaaatcaa a 41
<210> 17
   <211> 41
   <212> DNA
   <213> Homo Sapiens
<400> 17
   agtctgaatt tccagaaaac aatgatcatt caatggatca c 41
<210> 18
   <211> 41
   <212> DNA
   <213> Homo Sapiens
<400> 18
   atctccaagt caaagtttac ctcaagttgg gccaatccct g 41
Organization Applicant
Street: 30-36 Newport Road
City: Cardiff
State :
Country: United Kingdom
PostalCode : CF24 0DE
PhoneNumber:
FaxNumber:
EmailAddress :
<110> OrganizationName : University College Cardiff Consultants Limited
   Individual Applicant
**--------------------**
Street: MRC Centre for Neuropsychiatric Genetics & Genomics, Department of Psychological
Medicine & Neurology, School of Medicine, Cardiff University, Henry Wellcome Building, Heath Park,
City: Cardiff
State :
Country: United Kingdom
PostalCode : CF14 4XN
PhoneNumber:
FaxNumber:
EmailAddress :
<110> LastName : OWEN
<110> FirstName : Michael
<110> Middlelnitial : J
<110> Suffix : Prof
   Individual Applicant
--------------------
Street: Professor of Neuropsycological Genetics, Cardiff University, Department of Psychological
Medicine, GF41 Henry Wellcome Building, Heath Park,
City: Cardiff
State :
Country: United Kingdom
PostalCode : CF14 4XN
PhoneNumber:
FaxNumber :
EmailAddress :
<110> LastName : WILLIAMS
<110> FirstName : Julie
<110> Middlelnitial :
<110> Suffix : Prof
   Application Project
-------------------
<120> Title : Diagnosis of Alzheimer's Disease
<130> AppFileReference : UC3.67
<140> CurrentAppNumber : GB 0917326.1
   <141> CurrentFilingDate : 2009-10-03
   Earlier Applications
--------------------
<150> PriorAppNumber: GB0911539.5
   <151> PriorFilingDate : 2009-07-03
   Earlier Applications
--------------------
<150> PriorAppNumber: GB0913357.0
   <151> PriorFilingDate : 2009-07-31
   Sequence
<213> OrganismName : Homo Sapiens
<400> PreSequenceString :
   agccacacca gctatcaaaa ttctctaacg ggcccttgcc a 41
<212> Type : DNA
<211> Length : 41
   SequenceName : UC3.67 - SEQ ID 1
SequenceDescription :
Sequence
<213> OrganismName : Homo Sapiens
<400> PreSequenceString :
   gccatggaca tccacttcca tagcccggcc ttccagcacc c 41
<212> Type : DNA
<211> Length : 41
   SequenceName : UC3.67 - SEQ ID 2
SequenceDescription :
Sequence
<213> OrganismName : Homo Sapiens
<400> PreSequenceString :
   gttatgtgtg aagtcattta taatagatag tgttgataat a 41
<212> Type : DNA
<211> Length : 41
   SequenceName : UC3.67 - SEQ ID 3
SequenceDescription :
Sequence
<213> OrganismName : Homo Sapiens
<400> PreSequenceString :
   gttaacctgg gagtgaacta gacattaaag gggcagcata c 41
<212> Type : DNA
<211> Length : 41
   SequenceName : UC3.67 - SEQ ID 4
SequenceDescription :
Sequence
<213> OrganismName : Homo Sapiens
<400> PreSequenceString :
   aaatcaagaa gtgcatccat ctgattctga ataattggta c 41
<212> Type : DNA
<211> Length : 41
   SequenceName : UC3.67 - SEQ ID 5
SequenceDescription :
Sequence
<213> OrganismName : Homo Sapiens
<400> PreSequenceString :
   tctcagtagt ggggttttgt cacctttact gttattatta g 41
<212> Type : DNA
<211> Length : 41
   SequenceName : UC3.67 - SEQ ID 6
SequenceDescription :
Sequence
<213> OrganismName : Homo Sapiens
<400> PreSequenceString :
   tgcctgcagc ccaacagatg gcagtgtgct taacagctct g 41
<212> Type : DNA
<211> Length : 41
   SequenceName : UC3.67 - SEQ ID 7
SequenceDescription :
Sequence
<213> OrganismName : Homo Sapiens
<400> PreSequenceString :
   tataccccgt taaaggaaac gatatagaat acgaatggtc t 41
<212> Type : DNA
<211> Length : 41
   SequenceName : UC3.67 - SEQ ID 8
SequenceDescription :
Sequence
<213> OrganismName : Homo Sapiens
<400> PreSequenceString :
   tagtttcaag taaacatgtc gcagtgaagt ttgttgtaga g 41
<212> Type : DNA
<211> Length : 41
   SequenceName : UC3.67 - SEQ ID 9
SequenceDescription :
Sequence
<213> OrganismName : Homo Sapiens
<400> PreSequenceString :
   ccctgtccgt ccagactccg agagtctccg acgggtacta c 41
<212> Type : DNA
<211> Length : 41
   SequenceName : UC3.67 - SEQ ID 10
SequenceDescription :
Sequence
<213> OrganismName : Homo Sapiens
<400> PreSequenceString :
   caggctgcga actttgcacc tttacaccac tccacgtgac c 41
<212> Type : DNA
<211> Length : 41
   SequenceName : UC3.67 - SEQ ID 11
SequenceDescription :
Sequence
<213> OrganismName : Homo Sapiens
<400> PreSequenceString :
   caccacacac aaggcctgaa cgatcaaagg ctgaagagat g 41
<212> Type : DNA
<211> Length: 41
   SequenceName : UC3.67 - SEQ ID 12
SequenceDescription :
Sequence
<213> OrganismName : Homo Sapiens
<400> PreSequenceString :
   cgctccaaac ccgctgtgtc ataccatacc ggatgtttac c 41
<212> Type : DNA
<211> Length : 41
   SequenceName : UC3.67 - SEQ ID 13
SequenceDescription :
Sequence
<213> OrganismName : Homo Sapiens
<400> PreSequenceString :
   aatggtgaga taggtataga atagactgtc tcagttcaaa a 41
<212> Type : DNA
<211> Length: 41
   SequenceName : UC3.67 - SEQ ID 14
SequenceDescription :
Sequence
<213> OrganismName : Homo Sapiens
<400> PreSequenceString :
   ggcaaagagg ggaggaagat gccaatagct tagattccca 40
<212> Type : DNA
<211> Length : 40
   SequenceName : UC3.67 - SEQ ID 15
SequenceDescription :
Sequence
<213> OrganismName : Homo Sapiens
<400> PreSequenceString :
   gtttgggcat ctgggggaaa gccaggttta tgtaaatcaa a 41
<212> Type : DNA
<211> Length : 41
   SequenceName : UC3.67 - SEQ ID 16
SequenceDescription :
Sequence
<213> OrganismName : Homo Sapiens
<400> PreSequenceString :
   agtctgaatt tccagaaaac aatgatcatt caatggatca c 41
<212> Type : DNA
<211> Length : 41
   SequenceName : UC3.67 - SEQ ID 17
SequenceDescription :
Sequence
<213> OrganismName : Homo Sapiens
<400> PreSequenceString :
   atctccaagt caaagtttac ctcaagttgg gccaatccct g 41
<212> Type : DNA
<211> Length : 41
   SequenceName : UC3.67 - SEQ ID 18
SequenceDescription :

## Claims

1. A method for screening for, or diagnosing the likelihood of developing, or the existence of, Alzheimer's disease comprising:
a) providing a tissue sample which has been extracted from a human body of an individual to be tested wherein the tissue sample contains at least a locus containing the *PICALM* gene on chromosome 11;
b) examining said locus in order to identify whether SNP rs3851179 is present; and
c) where SNP rs3851179 is present concluding that the individual from whom the sample has been extracted is likely to develop, or is suffering from, Alzheimer's disease.

2. A method according to claim 1 comprising:
in step (a) additionally, providing a tissue sample that contains at least one locus selected from the following group the *complement receptor 1* gene *(CR1)* on chromosome 1, the *bridging integrator 1* gene (*BIN1*) on chromosome 2, *the ATP-binding cassette, sub-family A, member* 7 (*ABCA7*) on chromosome 19 and the *membrane-spanning 4A* (*MS4A*) gene cluster on chromosome 11;
in step (b) additionally examining said additional loci to see if any one or more of the following SNPs is present rs1408077, rs6701713 or rs3818361 in *CR1*; rs7561528 or rs744373 in *BIN1*; rs3764650 in *ABCA7,* and rs670139, rs610932, rs676309, rs667897, rs662196, rs583791 or rs1562990 in the *MS4A* gene cluster; and
in step (c) where the said one or more SNP is present concluding that the individual from whom the sample has been extracted is likely to develop, or is suffering from, Alzheimer's disease.

3. A method according to claim 1 wherein:
in step (b) additionally, said loci is examined to identify whether rs561655 or rs592297 is present; and
in step (c) where rs561655 or rs592297 is present concluding that the individual from whom the sample has been extracted is likely to develop, or is suffering from, Alzheimer's disease.

4. A method according to any one of the preceding claims wherein at least one labelled oligonucleotide, complementary to the genetic locus/loci or cluster to be examined, is used to detect said SNP(s), wherein said oligonucleotide upon binding to, and so detecting, said SNP, emits a detectable signal representative of the presence of said SNP.

5. A method according to any one of the preceding claims wherein said tissue sample is PCR amplified prior to performing step (b).

6. A method according to any one of the preceding claims wherein said tissue is enzymatically fragmented prior to performing step (b).

7. A method according to any one of claims 4-6 wherein said complementary oligonucleotide is attached or bound to a solid phase or substrate and said tissue sample is exposed to said solid phase prior to performing step (b).

8. A method for screening for, or diagnosing the likelihood of developing, or the existence of, Alzheimer's disease comprising:
a) providing a tissue sample which has been extracted from a human body of an individual to be tested wherein the tissue sample contains at least a locus containing *PICALM* gene on chromosome 11;
b) examining said locus in order to identify whether in *PICALM* SNP rs3851179 and/or SNP rs561655 or rs592297 which is in linkage disequilibrium therewith is present; and
c) where any one or more of the said SNPs is present concluding that the individual from whom the sample has been extracted is likely to develop, or is suffering from, Alzheimer's disease.

## Patentansprüche

1. Verfahren zum Screening auf oder Diagnostizieren der Wahrscheinlichkeit des Entwickelns oder des Vorliegens von Morbus Alzheimer, umfassend:
a) Bereitstellen einer Gewebeprobe, die von einem menschlichen Körper einer zu testenden Person extrahiert wurde, wobei die Gewebeprobe mindestens einen das *PICALM*-Gen auf Chromosom 11 enthaltenden Lokus enthält;
b) Untersuchen des Lokus zum Identifizieren dessen, ob SNP rs3851179 vorliegt; und
c) wenn SNP rs3851179 vorliegt, Rückschließen darauf, dass die Person, von welcher die Probe extrahiert wurde, wahrscheinlich Morbus Alzheimer entwickelt oder daran leidet.

2. Verfahren nach Anspruch 1, umfassend:
in Schritt (a) zusätzliches Bereitstellen einer Gewebeprobe, die mindestens einen Lokus enthält, der ausgewählt ist aus der folgenden Gruppe des *Komplement-Rezeptor*-*1*-Gens (*CR1*) auf Chromosom 1, des *Brückenintegrator-1*-Gens *(BIN 1*) auf Chromosom 2, der *ATP-Bindungskassette, Unterfamilie A, Vertreter* 7 *(ABCA7)* auf Chromosom 19 und des *membranüberspannenden 4A-* (*MS4A*-) -Genclusters auf Chromosom 11;
in Schritt (b) zusätzliches Untersuchen der zusätzlichen Loki, um zu sehen, ob irgendeine oder irgendwelche der folgenden SNPs vorliegen: rs1408077, rs6701713 oder rs3818361 in *CR1;* rs7561528 oder rs744373 in *BIN1;* rs3764650 in *ABCA7* und rs670139, rs610932, rs676309, rs667897, rs662196, rs583791 oder rs1562990 im *MS4A*-Gencluster; und
in Schritt (c), wenn der eine oder die mehreren SNP(s) vorliegen, Rückschließen darauf, dass die Person, von welcher die Probe extrahiert wurde, wahrscheinlich Morbus Alzheimer entwickelt oder daran leidet.

3. Verfahren nach Anspruch 1, wobei:
in Schritt (b) zusätzlich die Loki zum Identifizieren dessen untersucht werden, ob rs561655 oder rs592297 vorliegt; und
in Schritt (c), wenn rs561655 oder rs592297 vorliegt, Rückschließen darauf, dass die Person, von welcher die Probe extrahiert wurde, wahrscheinlich Morbus Alzheimer entwickelt oder daran leidet.

4. Verfahren nach einem der vorhergehenden Ansprüche, wobei mindestens ein markiertes Oligonukleotid, das zu dem/den zu untersuchenden genetischen Lokus/Loki oder Cluster, komplementär ist, zum Nachweisen des/der SNP(s) verwendet wird, wobei das Oligonukleotid durch Binden an und somit Nachweisen des SNP ein nachweisbares Signal aussendet, das für die Gegenwart des SNP repräsentativ ist.

5. Verfahren nach einem der vorhergehenden Ansprüche, wobei die Gewebeprobe vor dem Durchführen von Schritt (b) PCR-amplifiziert wird.

6. Verfahren nach einem der vorhergehenden Ansprüche, wobei die Gewebeprobe vor dem Durchführen von Schritt (b) enzymatisch gespalten wird.

7. Verfahren nach einem der Ansprüche 4-6, wobei das komplementäre Oligonukleotid an eine feste Phase oder ein festes Substrat angelagert oder gebunden ist und die Gewebeprobe vor dem Durchführen von Schritt (b) der festen Phase ausgesetzt wird.

8. Verfahren zum Screening auf oder Diagnostizieren der Wahrscheinlichkeit des Entwickelns oder des Vorliegens von Morbus Alzheimer, umfassend:
a) Bereitstellen einer Gewebeprobe, die von einem menschlichen Körper einer zu testenden Person extrahiert wurde, wobei die Gewebeprobe mindestens einen das *PICALM*-Gen auf Chromosom 11 enthaltenden Lokus enthält;
b) Untersuchen des Lokus zum Identifizieren dessen, ob in *PICALM* SNP rs3851179 und/oder damit in Bindungsungleichgewicht vorliegendes SNP rs561655 oder rs592297 vorliegt; und
c) wenn ein oder mehrere der SNPs vorliegen, Rückschließen darauf, dass die Person, von welcher die Probe extrahiert wurde, wahrscheinlich Morbus Alzheimer entwickelt oder daran leidet.

## Revendications

1. Procédé de criblage pour, ou de diagnostic de la susceptibilité de développer, ou de l'existence de, la maladie d'Alzheimer comprenant :
a) la fourniture d'un échantillon de tissu qui a été extrait d'un corps humain d'un individu à tester dans lequel l'échantillon de tissu contient au moins un locus contenant le gène *PICALM* sur le chromosome 11 ;
b) l'examen dudit locus afin d'identifier si le SNP rs3851179 est présent ; et
c) où le SNP rs3851179 est présent, la conclusion que l'individu duquel l'échantillon a été extrait est susceptible de développer, ou souffre de, la maladie d'Alzheimer.

2. Procédé selon la revendication 1 comprenant :
dans l'étape (a) en outre, la fourniture d'un échantillon de tissu qui contient au moins un locus choisi dans le groupe suivant : le gène *récepteur du complément 1* (*CR1*) sur le chromosome 1, le gène *intégrateur de pontage 1* (*BIN1*) sur le chromosome 2, la *cassette de liaison à l'ATP, sous-famille A, membre* 7 *(ABCA7)* sur le chromosome 19 et le groupe des gènes *transmembranaires 4A* (*MS4A*) sur le chromosome 11 ;
dans l'étape (b) en outre, l'examen desdits loci supplémentaires pour voir si l'un quelconque ou plusieurs des SNP suivants sont présents : rs1408077, rs6701713 ou rs3818361 *dans CR1 ;* rs7561528 ou rs744373 dans *BIN1 ;* rs3764650 dans ABCA7, et rs670139, rs610932, rs676309, rs667897, rs662196, rs583791 ou rs1562990 dans le groupe de gènes *MS4A* ; et
dans l'étape (c) où lesdits un ou plusieurs SNP sont présents, la conclusion que l'individu duquel l'échantillon a été extrait est susceptible de développer, ou souffre de, la maladie d'Alzheimer.

3. Procédé selon la revendication 1 dans lequel :
dans l'étape (b) en outre, lesdits loci sont examinés pour identifier si rs561655 ou rs592297 est présent ; et
dans l'étape (c) où rs561655 ou rs592297 est présent, la conclusion que l'individu duquel l'échantillon a été extrait est susceptible de développer, ou souffre de, la maladie d'Alzheimer.

4. Procédé selon l'une quelconque des revendications précédentes dans lequel au moins un oligonucléotide marqué, complémentaire du locus/des loci ou du groupe génétiques à examiner, est utilisé pour détecter le(s)dit(s) SNP, dans lequel ledit oligonucléotide après liaison, et ainsi détection, au dit SNP, émet un signal détectable représentatif de la présence dudit SNP.

5. Procédé selon l'une quelconque des revendications précédentes dans lequel ledit échantillon de tissu est amplifié par PCR avant d'effectuer l'étape (b).

6. Procédé selon l'une quelconque des revendications précédentes dans lequel ledit tissu est fragmenté par des enzymes avant d'effectuer l'étape (b).

7. Procédé selon l'une quelconque des revendications 4 à 6 dans lequel ledit oligonucléotide complémentaire est fixé ou lié à une phase solide ou un substrat et ledit échantillon de tissu est exposé à ladite phase solide avant d'effectuer l'étape (b).

8. Procédé de criblage pour, ou de diagnostic de la susceptibilité de développer, ou de l'existence de, la maladie d'Alzheimer comprenant :
a) la fourniture d'un échantillon de tissu qui a été extrait d'un corps humain d'un individu à tester dans lequel l'échantillon de tissu contient au moins un locus contenant le gène *PICALM* sur le chromosome 11 ;
b) l'examen dudit locus afin d'identifier si dans *PICALM* le SNP rs3851179 et/ou le SNP rs561655 ou rs592297 qui est en déséquilibre de liaison avec celui-ci est présent ; et
c) où l'un quelconque ou plusieurs desdits SNP sont présents, la conclusion que l'individu duquel l'échantillon a été extrait est susceptible de développer, ou souffre de, la maladie d'Alzheimer.
